# EUROPÄISCHE PATENTSCHRIFT

(11) **EP 2 297 252 B1**
(45) Veröffentlichungstag und Bekanntmachung des Hinweises auf die Patenterteilung: **04.03.2015**
(21) Anmeldenummer: 09753678.3
(22) Anmeldetag: 28.05.2009
(51) Int. Cl.: C09C 1/62, C09C 1/64, C09C 1/66, C09D 5/36, C09D 7/12, A61K 8/11, A61Q 1/02, A61K 8/39, A61Q 19/00, A61K 8/86, C08K 3/08, C08K 9/04

(54) **METALLEFFEKTPIGMENT MIT ADDITIV**
METAL EFFECT PIGMENT WITH ADDITIVE
PIGMENT À EFFET MÉTALLIQUE POURVU D'UN ADDITIF

(30) Priorität: 28.05.2008 EP 08009700
(43) Veröffentlichungstag der Anmeldung: 23.03.2011
(73) Patentinhaber: Eckart GmbH, 91235 Hartenstein (DE); BYK-Chemie GmbH, 46483 Wesel (DE)
(72) Erfinder: PRÖLSS, Dieter, 91126 Schwabach (DE); TRUMMER, Stefan, 90480 Nürnberg (DE); ROTH, Stephan, 95447 Bayreuth (DE); GERTZEN, Bärbel, 46446 Emmerich (DE); PRITSCHINS, Wolfgang, 46487 Wesel (DE); OMEIS, Jürgen, 46286 Dorsten-Lembeck (DE)
(74) Vertreter: Louis Pöhlau Lohrentz
(86) Internationale Anmeldenummer: PCT/EP2009/003824
(87) Internationale Veröffentlichungsnummer: WO 2009/144023

(56) Entgegenhaltungen:
- EP-A- 0 240 367
- EP-A- 0 569 907
- DE-A1- 10 361 437
- FR-A- 1 538 456
- FR-A- 2 064 024
- US-A- 3 551 174
- US-A- 3 857 865
- US-A- 4 484 951
- US-A- 4 629 512
- US-A1- 2007 051 272
- US-A1- 2008 087 187

## Beschreibung

Die vorliegende Erfindung betrifft ein Metalleffektpigment mit Additiv, ein Verfahren zu dessen Herstellung als auch dessen Verwendung sowie eine Beschichtungszusammensetzung, die das Metalleffektpigment mit Additiv enthält.

Metalleffektpigmente sind plättchenförmige Metallpigmente, die sich durch einen besonderen metallischen Glanz und Farbeffekte auszeichnen. Die Metalleffektpigmente werden in Farben, Lacken, Druckfarben, Kunststoffen, Kosmetika, etc. verwendet, da sie die Erzeugung von besonderen optischen Effekten, insbesondere Farb- und Glanzeffekte, ermöglichen.

Die Metalleffektpigmente werden herkömmlicherweise aus Metallgrieß durch Vermahlung in Kugelmühlen hergestellt. Der hierfür erforderliche Metallgrieß kann durch Zerstäubung von geschmolzenem Metall erhalten werden. Bei der Verformungsvermahlung des Metallgrießes werden gewöhnlich Schmiermittel zugegeben, um eine Kaltverschweißung der Metallpartikel miteinander zu vermeiden. Gewöhnlicherweise werden als Schmiermittel Ölsäure oder Stearinsäure verwendet. Dabei erzeugt Ölsäure non-leafing- und Stearinsäure leafing-Eigenschaften der vermahlenen Metalleffektpigmente. Statt dieser Fettsäuren können selbstverständlich auch jeweils höhere oder niedrigere Homologe wie beispielsweise Palmitinsäure als Schmiermittel verwendet werden. Fettsäuren von technischer Qualität bestehen regelmäßig aus einer Mischung verschiedenster homologer Fettsäuren, wobei bei einer gesättigten Fettsäure auch stets gewisse Anteile ungesättigter Fettsäuren und umgekehrt vorhanden sind. In der Praxis der Metalleffektpigmentherstellung ist es ebenfalls üblich, bewusst Mischungen von gesättigten und ungesättigten Fettsäuren, also beispielsweise eine Mischung aus Stearinsäure und Ölsäure einzusetzen.

Insbesondere bei der Verwendung von ungesättigten Fettsäuren als Schmierstoff stellt sich das Problem der Lagerstabilität von Metalleffektpigmenten. Die ungesättigten Fettsäuren neigen zur Polymerisation. Dies führt bei den Pigmenten, die meist als Pigmentpaste oder als Pigmentpulver gelagert werden, zu irreversiblen Agglomerationen.

Gemäß der DE 30 02 175 können auch Dicarbonsäuren als Schmiermittel verwendet werden.

Damit die Metalteffektpigmente in einem Anwendungsmedium, beispielsweise in einem Lack oder einer Farbe, optisch besonders ansprechend wirken, ist es erforderlich, dass die Metalleffektpigmente in dem Anwendungsmedium weitgehend planparallel orientiert sind, um das einfallende Licht gerichtet zu reflektieren. Eine statistische Ausrichtung der Orientierung der Metalleffektpigmente in dem Anwendungsmedium bewirkt eine ungerichtete Reflexion von einfallendem Licht in sämtliche Richtungen, was bei einem Betrachter zu einem geringwertigen optischen Eindruck führt.

Metalleffektpigmente stellen in einem Anwendungsmedium, beispielsweise einem Lack oder einer Farbe, einen Fremdkörper oder eine Störung dar, die zu verschlechterten mechanischen Eigenschaften, beispielsweise der getrockneten Farbe oder des gehärteten Lacks, führen können. Diese verschlechterten mechanischen Eigenschaften äußern sich beispielsweise in einer geringen Abriebfestigkeit, geringen Stabilität gegenüber Umwelteinflüssen wie Wärme, Kälte, Feuchtigkeit, etc.. Auch kann es zu einer Spaltung eines Druckfarben- oder Lackfilmes entlang der Ebene, in der die Metalleffektpigmente orientiert sind, und mithin zu großflächigen Ablösungen kommen.

Die WO 99/57204 offenbart mit Oberflächenmodifizierungsmitteln beschichtete Effektpigmente. Diese Oberflächenmodifizierungsmittel binden mit einer ersten reaktiven funktionellen Gruppe an die Pigmentoberfläche und mit wenigstens einer zweiten reaktiven funktionellen Gruppe, die von der ersten funktionellen Gruppe verschieden ist, beispielsweise an das Bindemittelsystem eines Lackes oder einer Farbe. Obgleich diese Oberflächenmodifizierung die mechanische Beständigkeit einer Lackschicht oder eines Farbfilmes erhöht, muß diese chemische Oberflächenmodifizierung in einem separaten Schritt durchgeführt werden, der zeit- und kostenaufwendig ist.

Die US 2007/0051272 A1 betrifft ein Verfahren zur Herstellung einer Metallflockenpigmentzusammensetzung. Die EP 0240367 A1 betrifft eine Aluminiumpigmentzusammensetzung. Die FR 2.064.024 offenbart ein Verfahren zur Herstellung einer Aluminiumpigmentzusammensetzung.

Die US 4,484,951 betrifft eine Aluminiumflockenzusammensetzung, die im Wesentlichen aus Aluminiumflockenpartikel und Dimersäure besteht.

Die DE 24 36 902 offenbart eine Estermasse, die Polyoxyalkylenglykolgruppen, monofunktionelle Alkoholgruppen sowie eine zweibasische Säure enthält, wobei der Ester eine Säurezahl von weniger als 25 und eine Hydroxylzahl von weniger als 25 aufweist und 2 bis 40 Gew.-% Polyoxyalkylenglykolgruppen enthält. Diese Estermasse wird in Form einer Emulsion mit Wasser als Schmiermittel bei der Bearbeitung von Eisen- und Nichteisenmetallen verwendet.

Die WO 2006/070108 A1 offenbart eine Metallpigmentzusammensetzung, bei deren Herstellung die Metallpartikel in Gegenwart eines Fettsäureesters R-COOR zerkleinert werden, wobei R ein Alkylrest mit 1 bis 8 C-Atomen ist.

Die WO 1998/17731 offenbart ein Verfahren zur Herstellung einer gering- oder nichtstaubenden Metalleffektpigmentzusammensetzung, wobei die Metallpartikel in Gegenwart von Korrosionsinhibitoren und einem Schmiermittel in einer wässerigen Flüssigkeit vermahlen werden. Als Korrosionsinhibitor können dabei organische Phosphorverbindungen, beispielsweise Phosphatester von langkettigen ethoxylierten Alkoholen verwendet werden.

Die EP 1 304 210 A1 offenbart Prozesshilfsmittel für die Verarbeitung von Kunststoffmassen. Das Prozesshilfsmittel enthält dabei Partialester von Polycarbonsäuren. Bei den Polycarbonsäuren kann es sich um Dimer- oder Trimersäuren mit 30 bis 60 C-Atomen handeln.

Im Stand der Technik werden Schmier- oder Prozeßhilfsmittel für die Be- oder Verarbeitung von Metallen oder Kunststoffen offenbart, wobei es keinen Bezug zur Herstellung oder Verwendung von Metalleffektpigmenten gibt.

Die bislang bei der Herstellung von Metalleffektpigmenten verwendeten Schmiermittel erlauben es nicht, nach Einarbeitung dieser Metalleffektpigmente in ein Anwendungsmedium eine Beschichtung mit verbesserten mechanischen Eigenschaften, insbesondere einer geringeren Spaltbarkeit von Farbschichten oder Lackfilmen zu erhalten, ohne dass zuvor eine aufwendige Oberflächenmodifizierung der Metalleffektpigmentoberfläche erfolgt ist.

Aufgabe der Erfindung ist es mithin, Metalleffektpigmente bereitzustellen, die einfach herzustellen und nachfolgend ohne aufwendige Nachbearbeitung in einem Anwendungsmedium verwendbar sind und eine verbesserte Lagerstabilität aufweisen. Die unter Verwendung des metalleffektpigmentierten Anwendungsmediums erhaltenen Beschichtungen sollen bei sehr guten optischen Eigenschaften wie hohem Glanz verbesserte mechanische Eigenschaften aufweisen.

Die der Erfindung zugrunde liegende Aufgabe wird durch Bereitstellung eines Metalleffektpigmentes mit Additiv gelöst, wobei das Additiv zumindest teilweise auf dem Metalleffektpigment aufgebracht ist und als Struktureinheiten wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind, wobei die Carbonsäure wenigstens eine Polycarbonsäure mit zwei bis acht Carbonsäuregruppen ist.

Bevorzugte Weiterbildung des erfindungsgemäßen Metalleffektpigmentes sind in den Unteransprüchen 2 bis 11 angegeben.

Die Aufgabe wird ferner durch Bereitstellung eines Verfahrens zur Herstellung des erfindungsgemäßen Metalleffektpigmentes gelöst, wobei das Verfahren die folgenden Schritte umfaßt:
a) Vermahlen von Metallpartikeln zu Metalleffektpigmenten in Gegenwart eines Additivs, das als Struktureinheiten wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind und wobei die Carbonsäure wenigstens eine Polycarbonsäure mit zwei bis acht Carbonsäuregruppen ist, und Mahlkörpern sowie optional einer Flüssigphase,
b) Abtrennen der in Schritt a) erhaltenen mit dem Additiv versehenen Metalleffektpigmente von den Mahlkörpern und optional der Flüssigphase,
c) optional Kompaktieren der in Schritt b) abgetrennten mit dem Additiv versehenen Metalleffektpigmente.

Bevorzugte Weiterbildungen des erfindungsgemäßen Verfahrens sind in den Unteransprüchen 13 und 14 angegeben.

Die Aufgabe wird ferner durch die Verwendung der erfindungsgemäßen Metalleffektpigmente zur Herstellung von Beschichtungszusammensetzungen, insbesondere Lacken, Coatings, Druckfarben, Kunststoffen oder kosmetischen Formulierungen als auch durch die Bereitstellung einer die erfindungsgemäße Metalleffektpigmente enthaltenden Beschichtungszusammensetzung gelöst.

Unter "Struktureinheiten" wird erfindungsgemäß verstanden, dass das Additiv eine Carbonsäure mit wenigstens 4 Kohlenstoffatomen umfaßt. Die Carbonsäure kann dabei als solche oder als Substituent, beispielsweise in Form einer Seitenkette, vorliegen. Wesentlich ist, dass das verwendete Additiv jedenfalls wenigstens eine Struktureinheit in Form einer Carbonsäure mit wenigstens 4 Kohlenstoffatomen aufweist.

Die Erfinder haben überraschend festgestellt, dass das erfindungsgemäß verwendete Additiv zu einer verbesserten Lagerstabilität von Pasten oder Pulvern der Metalleffektpigmente führt. Weiterhin führt das erfindungsgemäß verwendete Additiv zu einer verbesserten mechanischen Stabilität von aufgebrachten und getrockneten und/oder gehärteten Beschichtungszusammensetzungen, die die erfindungsgemäßen Metalleffektpigmente enthalten.

Darüber hinaus hat sich überraschenderweise gezeigt, dass ein Additiv, welches als Struktureinheiten wenigstens eine Polycarbonsäure mit wenigstens 4 Kohlenstoffatomen sowie wenigstens einen Polyglykolether umfaßt, wobei die Carbonsäure und der Polyglykolether kovalent miteinander verbunden sind, sich hervorragend als Schmiermittel eignet, welches bei der Herstellung der Metalleffektpigmente durch mechanische Vermahlung von Metallpartikel, bevorzugt Metallgrieß erforderlich ist. Überraschenderweise können die durch mechanische Vermahlung erhaltenen Metalleffektpigmente bis zu Metallplättchen mit äußerst geringen Dicken geformt werden, die einen hohen Glanz bzw. eine hohe Brillanz aufweisen.

Bei einer ebenfalls bevorzugten Ausführungsform eignet sich das Additiv sehr gut als Dispergieradditiv für Metalleffektpigmente. Dabei sind besonders bevorzugt eine Metalleffektpigmentpaste und/oder ein Metalleffektpigmentfilterkuchen, denen dieses Additiv zugesetzt wird. Derartige Pasten weisen eine signifikant erhöhte Lagerstabilität auf.

Unter einer erhöhten "Lagerstabilität" wird verstanden, dass die Metalleffektpigmente während der Lagerung nicht oder nur unwesentlich agglomerieren. Insbesondere tritt keine oder nur eine unwesentliche Agglomeration bei Lagerung der erfindungsgemäßen Metalleffektpigmente in komprimierter Form, insbesondere als Paste oder als Pulver, auf.

Gemäß einer Variante der Erfindung weist ein signifikanter Anteil der Metalleffektpigmentoberfläche das erfindungsgemäß zu verwendende Additiv auf. Gemäß einer bevorzugten Ausführungsform sind wenigstens 30 %, weiter bevorzugt wenigstens 50 %, noch weiter bevorzugt wenigstens 80 %, noch weiter bevorzugt wenigstens 90 %, der Metalleffektpigmentoberfläche mit dem erfindungsgemäß zu verwendenden Additiv versehen.

Bevorzugt sind 0,2 bis 5 Gew.-% Additiv weiter bevorzugt 0,4 bis 4 Gew.-% und besonders bevorzugt 0,5 bis 3 Gew.-% Additiv, jeweils bezogen auf die Menge an Metalleffektpigment und Additiv, auf der Oberfläche des Metalleffektpigmentes gebunden.

Dabei hängt das Mengenverhältnis des Additivs zum Metalleffektpigment vor allem von der Art des Metalls und der spezifischen Oberfläche des Metalls ab.

Um hiervon unabhängig zu sein, lässt sich die Menge an Additiv (in ng) auch als Menge Additiv bezogen auf 1 cm² Pigmentoberfläche darstellen.
Dabei sind bevorzugt 10 bis 3.000 ng/cm², weiter bevorzugt 30 bis 2.000 ng/cm² und besonders bevorzugt 50 bis 1.000 ng/cm² Additiv auf der Oberfläche des Metalleffektpigmentes gebunden.

Bei Anwendungen im Druckbereich ergibt sich ein weiterer Vorteil:
Dispergieradditive, die einer Metalleffektpigmente enthaltenen Druckfarbe zugesetzt werden, sind gewöhnlicherweise zu einem weitaus geringeren Anteil auf der Metallpigmentoberfläche gebunden. Häufig wirkt sich dies jedoch störend aus, da viele Dispergieradditive als Schaumbildner wirken und mithin ungewollt viel Schaum beim Einarbeiten des Metalleffektpigmentes in die Druckfarbe entsteht. Die erfindungsgemäßen Metalleffektpigmente weisen diesen Nachteil hingegen nicht auf, da das Additiv in einer bevorzugten Ausführungsform bereits während der Vermahlung zugesetzt wird und mithin an die Metalleffektpigmentoberfläche gebunden ist. Somit kann bei der Einarbeitung der erfindungsgemäßen Metalleffektpigmente in eine Druckfarbe der Anteil an separat zuzusetzendem Dispergieradditiv verringert werden. Gemäß einer bevorzugten Variante der Erfindung kann auf die zusätzliche Verwendung von Dispergieradditiv in der Druckfarbe verzichtet werden.

Obgleich bislang noch nicht geklärt ist, warum das erfindungsgemäß verwendete Additiv auf der einen Seite eine bei der mechanischen Vermahlung von Metallgrieß zu Metalleffektpigmenten sehr gute Schmierwirkung aufweist und zum anderen eine verbesserte Einfügung oder Einbindung der so hergestellten Metalleffektpigmente in den Anwendungsmedien, wie Farben, Druckfarben, Lacke, etc. ermöglicht, wird vermutet, ohne dass die Erfinder dabei an diese Vermutung gebunden sein wollen, dass die sehr gute Schmierwirkung durch die Carbonsäure mit wenigstens 4 Kohlenstoffatomen zusammen mit dem Polyglykolether erzeugt wird.

Es wird weiterhin vermutet, dass die Schmierwirkung durch einen synergistischen Effekt zwischen Carbonsäure und Polyglykolether verstärkt wird. Hier könnte durch die kovalente Bindung von Carbonsäure und Polyglykolether eine vorteilhafte räumliche Nähe erzeugt werden, die für sehr gute Schmierwirkung des Additivs von Bedeutung sein könnte.

Die gute Einfügung oder Einbindung in ein Anwendungsmedium sowie die hohe Lagerstabilität könnte ebenfalls auf das gleichzeitige Vorhandensein und die enge räumliche Kopplung von Carbonsäure und Polyglykolether zurückzuführen sein. Die Carbonsäure(n) sind vom Charakter eher hydrophobe Moleküle, die eine große Affinität zu unpolareren organischen Lösemitteln aufweisen. Die gilt insbesondere bei längerkettigen Carbonsäuren. Die Polyglykolether sind im Hinblick auf die in den Kohlenwasserstoffketten enthaltenen Sauerstoffatome eher polarer Natur und mithin hydrophile Moleküle. Sie besitzen eine sehr gute Löslichkeit in einer Vielzahl von polaren und auch unpolaren Solventien. Insbesondere sind sie gut wasserlöslich und sind daher auch oft in Tensiden für wässrige Anwendungen zu finden. Durch die Kombination von hydrophoben und hydrophilen Eigenschaften können die erfindungsgemäßen Metalleffektpigmente sowohl mit hydrophoben als auch mit hydrophilen Komponenten von Anwendungsmedien in Wechselwirkung treten, weshalb die erfindungsgemäßen Metalleffektpigmente vermutlich sehr gut von Farben, Lacken, Druckfarben, etc. umhüllt und mithin in das Anwendungsmedium eingefügt oder eingebunden werden, ohne dabei als wesentliche Störung oder als Fremdkörper zu wirken. Die gute Einfügung oder Einbindung in das Anwendungsmedium könnte dann zu der festgestellten verbesserten mechanischen Beständigkeit in einer Farbschicht, Lackschicht, Druckfarbschicht, etc. führen. Diese Eigenschaften gelten überraschenderweise sowohl für wasser- wie auch für lösemittel basierende Anwendungssysteme.

Gemäß einer bevorzugten Weiterbildung der Erfindung werden die Metalle der Metalleffektpigmente aus der Gruppe, die aus Aluminium, Kupfer, Zink, Zinn, Goldbronze, Eisen, Titan, Chrom, Nickel, Silber, Gold, Stahl sowie Legierungen davon und Gemischen dieser Metalle besteht, ausgewählt werden.

Äußerst bevorzugt werden Metalleffektpigmente mit oder aus Aluminium, Eisen, Kupfer oder Goldbronze verwendet. Goldbronze steht hier für Messing, d.h. eine Legierung aus Kupfer und Zink.

Die mittlere Dicke h₅₀ der erfindungsgemäßen Metalleffektpigmente liegt vorzugsweise in einem Bereich von 15 nm bis 5 µm, weiter bevorzugt von 20 nm bis 2 µm, noch weiter bevorzugt von 30 nm bis 1 µm. Als sehr geeignet hat sich auch ein Dickenbereich von 50 nm bis 500 nm oder von 70 nm bis 150 nm erwiesen.

Bei einer besonders bevorzugten Ausführungsform werden die Metalleffektpigmente durch Vermahlung hergestellt.
Die erfindungsgemäßen Metalleffektpigmente können prinzipiell sogenannte "Cornflakes" oder auch sogenannte "Silberdollars" sein. Unter Cornflakes versteht man Metalleffektpigmente, die hauptsächlich durch Zerkleinerungsmahlung erhalten werden und die ausgefranste Ränder und eine relativ raue Oberfläche aufweisen. Die optisch höherwertigen Silberdollarpigmente werden hauptsächlich durch Verformungsmahlung erhalten und weisen runde Ränder und wesentlich glattere Oberflächen auf. Aus diesem Grund weisen sie weniger Streuzentren auf, was zu einem erhöhtem Glanz und Hell-Dunkelflop führt.

In den letzten Jahren sind weitere, sehr dünne Aluminiumeffektpigmente beschrieben worden, die zunehmend die bisher existierende Kluft zwischen den optisch sehr hochwertigen PVD-Pigmenten und den konventionell vermahlenen Pigmenten andererseits schließen sollen.

So sind in der EP 1 621 586 A1 Aluminiumpigmente mit Dicken von 20 bis 80 nm beschrieben worden. Die WO 2004/087816 A2 beschreibt ebenfalls dünne Aluminiumpigmente mit mittleren Dicken von 30 bis 100 nm, die zudem eine sehr glatte Oberfläche und eine enge Dickenverteilung der Pigmente aufweisen.

Völlig überraschenderweise können die erfindungsgemäßen Metalleffektpigmente aufgrund der hervorragenden Schmierwirkung des erfindungsgemäß zu verwendenden Additivs sehr leicht bis zu sehr geringen Pigmentdicken ausgeformt werden. Dies ist keineswegs nur auf Aluminiumeffektpigmente beschränkt, vielmehr können auch sehr dünne Goldbronze- oder Eisenpigmente erhalten werden.

Erfindungsgemäß bevorzugt sind bei Aluminium-, Goldbronze- und/oder Eiseneffektpigmenten sehr dünne Metalleffektpigmente mit einer mittleren Dicke h₅₀ von 15 bis 100 nm, besonders bevorzugt von 17 bis 80 nm und ganz besonders bevorzugt von 20 nm bis 50 nm.

Die mittlere Größe der Metalleffektpigmente liegt vorzugsweise in einem Bereich von 1 µm bis 200 µm, weiter bevorzugt von 3 µm bis 150 µm und noch weiter bevorzugt von 5 µm bis 100 µm.

Die Metalleffektpigmente sind plättchenförmig, so dass das mittlere Größen-Dicken-Verhältnis (Formfaktor) bevorzugt wenigstens 5 :1, vorzugweise wenigstens 10 :1, weiter bevorzugt wenigstens 20 :1, noch weiter bevorzugt wenigstens 50 :1 beträgt.

Als sehr geeignet hat sich ein auch ein Größen-Dicken-Verhältnis von 100 :1 oder 1.500 :1 erwiesen

Bei einer weiteren bevorzugten Ausführungsform handelt es sich bei den Metalleffektpigmenten um mittels des PVD-Verfahren hergestellte Metalleffektpigmente. Hierbei sind insbesondere Aluminiumeffektpigmente besonders bevorzugt.

Naturgemäß wird bei diesen Ausführungsformen das Additiv nicht als Schmiermittel verwendet, sondern bevorzugt der vorliegenden Metalleffektpigmentdispersion zugesetzt.

Die erfindungsgemäßen Metalleffektpigmente besitzen vorzugsweise aufgrund der möglichen sehr geringen mittleren Dicken eine sehr hohe Deckkraft. Als Deckkraft oder Deckvermögen eines Pigmentes wird üblicherweise die Abdeckung einer Fläche pro Gewichtseinheit an Pigmentmenge bezeichnet. Je dünner die mittlere Dicke der Metalleffektpigmente ist, desto größer ist die durch das Pigment abgedeckte Fläche (pro Gewichtseinheit des Metalleffektpigmentes) und mithin die Deckkraft des Metalleffektpigmentes.

Eine geringe Dicke ist insbesondere in Drucken von sehr großem Vorteil, da diese, beispielsweise im Vergleich zu Lackbeschichtungen, eine wesentlich geringere Dicke und einen geringeren Bindemittelanteil aufweisen.

Die erfindungsgemäßen Metalleffektpigmente sind mithin sehr geeignet zur Verwendung in Druckfarben. Das erfindungsgemäß zu verwendende Additiv bewirkt eine verbesserte Einfügung des erfindungsgemäßen Metalleffektpigmentes in die verdruckte Druckfarbschicht.

Die erfindungsgemäßen Metalleffektpigmente zeichnen sich durch eine außerordentliche Brillanz und einem hervorragenden spezifischen Deckvermögen aus. Insbesondere bei Druckapplikationen ergeben sich brillante Farbtöne. Beispielsweise lassen sich im Falle von erfindungsgemäßen Aluminiumeffektpigmenten hochglänzende silberfarbene Beschichtungen erzeugen.

Bei Verwendung von erfindungsgemäßen Messing- oder Goldbronzeeffektpigmenten werden hochglänzende goldfarbene Beschichtungen erzeugt.

Bei Aufbringung der Metalleffektpigmente auf Folien, beispielsweise Kunststofffolien, in Form von Rückseitenapplikationen werden Folien mit außerordentlicher metallischer Brillanz bereitgestellt, insbesondere wenn sie von der Vorderseite betrachtet werden.

Diese Rückseitenapplikationen der erfindungsgemäßen Metalleffektpigmente auf Folienmaterial eignen sich insbesondere für flexible Verpackungen, Schrumpffolien, Laminate (z.B. auf Karton), Verpackungsfolien, Etiketten, InMouldDecoration-Folien im Siebdruck (z.B: für Handy-Oberschalen), etc..

Die erfindungsgemäßen Metalleffektpigmente werden vorzugsweise in Druckfarben eingearbeitet und sodann auf herkömmlichen Druckmaschinen auf Papier, Karton, Folien, Textilien, etc. verdruckt.

Bei einer bevorzugten Ausführungsform wird durch die mechanische Vermahlung des Metallpartikels in Gegenwart des erfindungsgemäß zu verwendenden Additivs das Additiv auf die entstehende Metalleffektpigmentoberfläche aufgebracht. Das Additiv kann dabei durch physikalische Effekte und/oder durch chemische Bindung auf die Metalleffektpigmentoberfläche gebunden werden.

Gemäß einer bevorzugten Ausführungsform werden die erfindungsgemäßen Metalleffektpigmente nach der mechanischen Vermahlung in Gegenwart des erfindungsgemäß zu verwendenden Additivs nicht weiter beschichtet. Die erfindungsgemäßen Metalleffektpigmente können mithin, gegebenenfalls unter Wechsel oder Entfernung des Lösemittels, direkt in ein Applikationsmedium eingearbeitet werden.

Selbstverständlich können die erfindungsgemäßen Metalleffektpigmente auch weiter beschichtet und somit die Metalleffektpigmentoberfläche organisch-chemisch modifiziert werden.

Gemäß einer bevorzugten Ausführungsform der Erfindung sind die Metalleffektpigmente non-leafing-Pigmente. Unter non-leafing Metalleffektpigmenten wird verstanden, dass sich die Metalleffektpigmente nicht an oder in der Nähe der Oberfläche eines Anwendungsmediums, d.h. auf der dem Substrat abgewandten Fläche des Anwendungsmediums, beispielsweise eines Lack-, Farb- oder Druckfarbenfilms, sondern sich in dem Anwendungsmedium anordnen. Die erfindungsgemäßen Metalleffektpigmente werden mithin von dem Anwendungsmedium, beispielsweise Bindemittel, umhüllt und bei Trocknung oder Aushärtung umschlossen oder eingebunden. Somit sind die erfindungsgemäßen non-leafing Metalleffektpigmente durch den Lack, die Farbe oder Druckfarbe bereits vor mechanischer oder chemischer Einwirkung geschützt.

Selbstverständlich kann auf eine Beschichtung mit erfindungsgemäßen Metalleffektpigmenten auch noch eine Schutzschicht, beispielsweise ein Klarlack, aufgebracht werden.

Den erfindungsgemäßen Metalleffektpigmenten kann auch leafing-Verhalten verliehen werden, beispielsweise indem auf die Pigmentoberfläche zusätzlich gesättigte Fettsäuren mit mindestens 12 C-Atomen, bevorzugt Palmitinsäure oder Stearinsäure, aufgebracht wird.

Das erfindungsgemäß zu verwendende Additiv ist durch Umsetzung von Carbonsäure und Polyglykolether erhältlich.
Dabei findet die Unsetzung von Carbonsäure und Polyglykolether bevorzugt durch Veresterung und/oder Amidierung statt.

Besonders bevorzugt erfolgt dabei die kovalente Verknüpfung von Polyglykolether mit der Carbonsäure durch eine Veresterung. Es werden hierbei die Carbonsäurefunktionen ganz oder zumindest teilweise mit dem Polyglykolether verestert.

Beispielsweise können Carbonsäure und Polyglykol durch herkömmliche Veresterungsreaktion, beispielsweise durch Temperaturerhöhung und Wasserentzug, miteinander umgesetzt werden. Die Bedingungen solcher Veresterungsreaktionen sind dem Fachmann bekannt und auch beispielsweise in der EP 1 304 210 A1 oder der DE 24 36 902 beschrieben, die hiermit unter Bezugnahme aufgenommen sind.

Die Veresterungsgrade liegen hierbei bevorzugt bei 10 % bis 90 %, weiter bevorzugt bei 20 % bis 80 % und besonders bevorzugt bei 25 % bis 75 %.
Innerhalb dieser Bereiche erhält man eine befriedigende Balance zwischen den hydrophoben und hydrophilen Komponenten des Additivs.

Gemäß einer weiteren Ausführungsform ist die Carbonsäure gesättigt oder ungesättigt. Dabei sind jedoch gesättigte Carbonsäuren bevorzugt, da sie eine längere Lagerstabilität bewirken.

Die Carbonsäure weist bevorzugt 6 bis 130 Kohlenstoffatome, weiter bevorzugt 8 bis 100 Kohlenstoffatome, besonders bevorzugt 10 bis 96 Kohlenstoffatome, und ganz besonders bevorzugt 20 bis 80 Kohlenstoffatome auf. Dabei bezieht sich diese Anzahl der Kohlenstoffatome auf das Kohlenwasserstoffgrundgerüst der Carbonsäuren einschließlich der Carboxylatfunktionen, jedoch nicht auf die Polyglykohlethereinheiten.

Unterhalb von 4 Kohlenstoffatomen in der Carbonsäurestruktureinheit, vorzugsweise Carbonsäure oder Carbonsäurerest, sind die vorteilhaften Wirkungen des Additivs in Verbindung mit dem Metallpigment nicht erkennbar. Oberhalb von 130 C-Atomen wird das Additiv zunehmend in den meisten Lösemitteln unlöslich. Es kann daher nur schwer synthetisiert werden und zeigt ebenfalls kaum noch die vorteilhaften Wirkungen in Verbindung mit dem Metalleffektpigment.

Als bei der vorliegenden Erfindung geeignete Monocarbonsäure mit wenigstens 4 Kohlenstoffatomen, die mit Polyglykolether kovalent verbunden werden können, haben sich gesättigte Fettsäuren erwiesen. Vorzugsweise werden Fettsäuren mit 6 bis 30 Kohlenstoffatomen, weiter bevorzugt mit 10 bis 24 Kohlenstoffatomen, noch weiter bevorzugt mit 14 bis 22 Kohlenstoffatomen verwendet. Es können auch Mischungen verschiedener Monocarbonsäuren verwendet werden, in diesem Fall sind die vorstehend genannten Werte für die Anzahl der Kohlenstoffatome als Mittelwerte der Mischung mehrerer Monocarbonsäuren zu verstehen.

Vorzugsweise werden die Fettsäuren aus der Gruppe ausgewählt, die aus Valeriansäure, Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure, Melissinsäure und Mischungen dieser Fettsäuren besteht.

Als Monocarbonsäuren mit wenigstens 4 Kohlenstoffatomen können erfindungsgemäß auch ungesättigte Fettsäuren verwendet werden. Vorzugsweise werden ungesättigte Fettsäuren mit 6 bis 30 Kohlenstoffatomen, weiter bevorzugt mit 10 bis 24 Kohlenstoffatomen, noch weiter bevorzugt mit 14 bis 22 Kohlenstoffatomen verwendet.

Die ungesättigten Fettsäuren können beispielsweise aus der Gruppe ausgewählt werden, die aus Undecylensäure, Palmitoleinsäure, Ölsäure, Elaidinsäure, Vaccensäure, Icosensäure, Cetoleinsäure, Erucasäure, Nervonsäure, Sorbinsäure, Linolsäure, Linolensäure, Elaeostearinsäure, Arachidonsäure, Timnodonsäure, Clupanodonsäure, Docosahexaensäure und Mischungen dieser Fettsäuren besteht.

Gemäß einer weiteren bevorzugten Ausführungsform werden als Carbonsäuren Dicarbonsäuren, Tricarbonsäuren, Tetracarbonsäuen oder deren Mischungen verwendet. Als Di- und/oder Tricarbonsäuren, die mit Polyglykolether kovalent verbunden werden können, können ebenfalls gesättigte und/oder ungesättigte Carbonsäuren verwendet werden.

Beispielsweise können als Dicarbonsäuren Bernsteinsäure, Glutarsäure, Adipinsäure, Pimelinsäure, Korksäure, Azelainsäure und/oder Sebacinsäure verwenden werden.

Gemäß einer besonders bevorzugten Weiterbildung der Erfindung werden Di-, Tri- oder Tetracarbonsäuren mit längeren Kohlenstoffgerüsten verwendet. Diese Di-, Tri- oder Tetracarbonsäuren werden bevorzugt durch Di-, Tri- oder Tetramerisierung von ungesättigten Fettsäuren erhalten. Die dafür verwendeten Fettsäuren weisen bevorzugt 11 bis 30 Kohlenstoffatome, weiter bevorzugt 12 bis 24 Kohlenstoffatome und, noch weiter bevorzugt 14 bis 22 Kohlenstoffatome auf. Zur Di-, Tri- oder Tetramerisierung geeignete ungesättigter Fettsäuren sind beispielsweise Ölsäure, Linolsäure, Linolensäure, Eleostearinsäure oder ähnlichen Säuren.
Obgleich unverzweigte Fettsäuren bevorzugt verwendet werden, können selbstverständlich auch verzweigte Fettsäuren verwendet werden.

Derartige Verbindungen werden als Naturprodukte erhalten und stellen bevorzugt Mischungen der Mono-, Di-, Tri- oder Tetracarbonsäuren oder höherer Homologe dar. Besonders bevorzugt liegen die Polycarbonsäuren überwiegend in der Form der Dicarbonsäure vor.

Als sehr geeignet haben sich Dicarbonsäuren mit einem Kohlenstoffgrundgerüst von 18 Kohlenstoffatomen erwiesen. Diese Dicarbonsäure weist demnach 36 C-Atome und die entsprechende Tricarbonsäure 54 C-Atome auf.

Die Verwendung von Dicarbonsäuren bei der Umsetzung mit Polyglykol und/oder Polyglykolether ist bevorzugt. Vorzugsweise erfolgt dabei nur eine Teilveresterung unter Erhalt von überwiegend Dicarbonsäuremonoglykolestern. Ein Dicarbonsäuremonoglykolester kann über die freie Carboxylatgruppe an die Metalleffektpigmentoberfläche binden. Bei dieser Bindung handelt es sich vorzugsweise um eine kovalente Bindung.

Vorzugsweise handelt es sich bei der Polycarbonsäure um eine monomere, dimerisierte, trimerisierte oder tetramerisierte Fettsäure. Bei den Fettsäuren können die oben genannten Fettsäuren verwendet werden, die dimerisiert, trimerisiert oder tetramerisiert werden. Vorzugsweise werden Mischungen dieser verschiedenen Fettsäuren verwendet.

Erfindungsgemäß ist die Carbonsäure wenigstens eine Polycarbonsäure mit zwei bis acht Carbonsäuregruppen. Weiter bevorzugt enthält die Polycarbonsäure 2 bis 4 Carboxylgruppen. Diese Angaben beziehen sich im Fall von Mischungen unterschiedlichster Poycarbonsäuren auf die Mittelwerte.

Weiterhin ist bevorzugt, dass die Polycarbonsäure 10 bis 96, vorzugsweise 12 bis 76, Kohlenstoffatome, weiter bevorzugt 24 bis 60 Kohlenstoffatome, noch weiter bevorzugt 36 bis 54 Kohlenstoffatome, enthält. Auch hier bezieht sich die Anzahl der Kohlenstoffatome im Fall von Mischungen verschiedenster Polycarbonsäuren auf die durchschnittliche Anzahl innerhalb dieser Mischung.

Vorzugsweise wird eine dimerisierte oder trimerisierte Fettsäure verwendet, die vorzugsweise 30 bis 60 Kohlenstoffatome, weiter bevorzugt 36 bis 54 Kohlenstoffatome aufweist. Als sehr geeignet hat sich eine Dimersäure mit durchschnittlich 36 Kohlenstoffatomen erwiesen. Die Dimersäure kann dabei zu gewissen Anteilen bevorzugt auch Trimersäure oder Monosäure oder Tetramersäure enthalten.

Entsprechende Polycarbonsäuren sind kommerziell erhältlich unter den Handelsnamen Empol (Fa. Cognis, Adhesives & Sealants) oder Pripol (Fa. Unichema) oder Versadyne (Henkel Hakusui Kabushiki Kaisha).
Beispiele hierfür sind: Empol 1018, Empol 1045, Pripol 1013, Pripol 1006, Pripol 1022, Pripol 1009, Pripol 1010, Pripol 1040, Pripol 1010 oder Versadyme 216.

Gemäß einer bevorzugten Weiterbildung der Erfindung, umfasst der Polyglykolether die Gruppe R¹-X-(R²-O)_{y}-(R³-O)_{z}-(R⁴-O)ₖ-, wobei die R²-O-, R³-O- und die R⁴-O-Polyethereinheiten statistisch, alternierend oder als Blockcopolymere angeordnet sein können.

Der Rest R¹ ist ein linearer oder verzweigter aliphatischer Rest oder araliphatischer oder aromatischer organischer Rest mit 1 bis 30 Kohlenstoffatomen.
Die Reste R², R³ und R⁴ können gleich oder unabhängig voneinander verschieden sein und stehen jeweils für einen linearen oder verzweigten aliphatischen organischen Rest oder araliphatischen oder aromatischen organischen Rest mit 1 bis 12 Kohlenstoffatomen.

Die Einzelpolymerisationsgrade y, z und k sind natürliche Zahlen und stehen unabhängig voneinander für 0 bis 200, mit der Maßgabe das y+z+k = 2 bis 600 ist.

Die Gruppe X steht für O, S, (CO)O, NR^{x}, wobei R^{x} gleich H oder ein aliphatischer Rest mit 1 bis 20 Kohlenstoffatomen ist. Bevorzugt ist hierbei X ein Sauerstoffatom oder eine Carboxyfunktion und besonders bevorzugt ein Sauerstoffatom.

Der zur kovalenten Verknüpfung mit der Carbonsäure verwendete Polyglykolether wird bevorzugt erhalten durch Umsetzung eines Alkohols R¹-OH, eines Thiols R¹-SH, einer Carbonsäure R¹-COOH oder eines Amins R¹NHR als Startermoleküle mit jeweils einem Überschuß an Glykolen unter geeigneten, dem Fachmann bekannten Reaktionsbedingungen.

Die erfindungsgemäßen Polyglykolether liegen weitgehend als monofunktionelle Polyglykole vor, da diese sich in eindeutiger Weise sich mit den Carbonsäuren kovalent verknüpfen lassen. Unter "weitgehend monofunktionell" wird hier verstanden, dass sie einen Anteil von 0 bis maximal 10 % auch an bifunktionellen Polyglykolether aufweisen. In diesem Fall enthält entweder der Rest R¹ eine mit der Carbonsäure reaktionsfähige Gruppe oder statt des Restes R¹ ist einfach ein Wasserstoffatom vorhanden. Der letztere Fall beispielsweise ist auf eine unvollständige Umsetzung der Alkohole, Thiole usw. mit den Glykolen zurückzuführen. I

Der Rest R¹ ist bevorzugt ein linearer oder verzweigter aliphatischer Rest oder araliphatischer oder aromatischer organischer Rest mit 2 bis 16 Kohlenstoffatomen und besonders bevorzugt ein aliphatischer Rest mit 1 bis 12 C-Atomen.

Die Reste weisen R², R³ und R⁴ bevorzugt unabhängig voneinander 2 bis 8 C-Atome und besonders bevorzugt 2 bis 4 C-Atome auf.

Besonders bevorzugt handelt es sich bei den Resten R², R³ und R⁴ unabhängig voneinander um Ethyl, Iso-Propyl, Propyl oder Butyl. Weiter besonders bevorzugt sind alternierende oder blockcopolymerartige Ethyl-, Iso-Propyl-Einheiten, sogenannte EO/PO- Polyether.

Die Länge der Ethereinheiten y+z+k ist bevorzugt 5 bis 300, weiter bevorzugt 7 bis 100 und besonders bevorzugt 10 bis 50.

Bei zu langen Ethereinheiten nimmt die Affinität der Additive zur Metalleffektpigmentoberfläche ab. Somit besteht die Gefahr, dass insbesondere in einer Paste oder einem fertigen Applikationsmedium, beispielsweise einer Druckfarbe, das Additiv sich vom Metalleffektpigment löst bzw. gar nicht in ausreichender Menge an die Metalleffektpigmentoberfläche bindet. Sind hingegen die Ethereinheiten zu kurz, so sind die Additive in ihrer Wirkung auf die Eigenschaften der Metalleffektpigmente kaum oder nicht mehr von den bekannten Schmiermitteln zu unterscheiden.

Beispiele geeigneter Polyglykolether sind Methoxypolyethylenglycole, Butoxypolyethylenglycole, Methoxypolypropylenglycole oder Butoxypolypropylenglycole.

Vor der Ankopplung an die Carbonsäure weist der Polyglykolether bzw. das Polyglykol üblicherweise ein Wasserstoffatom oder eine Aminfunktion oder ein Epoxid an dem offenen Ende der vorstehend gezeigten Strukturformeln auf. Mithin sind vor der Umsetzung mit der Carbonsäure folgende Moleküle bevorzugt:

R¹-X-(R²-O)_{y}-(R³-O)_{z}-(R⁴-O)ₖ-H (Bildung eines Esters)

R¹-X-(R²-O)_{y}-(R³-O)_{z}-(R⁴-O)ₖ-N(H)(R⁵)R (Bildung eines Amids)

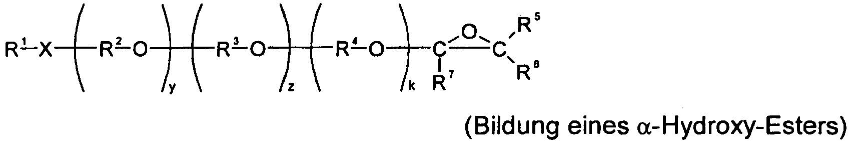

Bei den vorstehenden Strukturformeln sind R⁵, R⁶ und R⁷ unabhängig voneinander vorzugsweise H oder ein verzweigter oder unverzweigter Kohlenstoffrest mit 1 bis 6 Kohlenstoffatomen. Vorzugsweise ist der Kohlenstoffrest ein linearer Alkylrest mit 1 bis 6 Kohlenstoffatomen. Die Kohlenstoffreste R⁵, R⁶ und R⁷ können unabhängig voneinander gesättigt oder ungesättigt sein. Beispielsweise können R⁵, R⁶ und R⁷ unabhängig voneinander für Phenylrest stehen. Besonders bevorzugt sind R⁷ und R⁵ ein H und R⁶ ein H oder Methyl.

Nach Umsetzung des Polyglykolethers mit der Carbonsäure erfolgt vorzugsweise eine kovalente Kopplung über das endständige Sauerstoffatom.

Gemäß einer weiteren Variante ist die Carbonsäure teilweise oder vollständig verestert bzw. amidiert.

Es hat sich überraschend gezeigt, das es besonders vorteilhaft ist, wenn nur ein Teil der Carboxylgruppen verestert ist, mithin die Carbonsäure(n), vorzugsweise Polycarbonsäure(n), als Partialester vorliegen. Vorzugsweise sind mindestens 10 % und maximal 90 % der Carboxylgruppen verestert. Weiterhin bevorzugt sind etwa 15 bis 80 %, noch weiter bevorzugt etwa 20 bis 70 % der Carboxylgruppen verestert.

Zur Kopplung an die Metalleffektpigmentoberfläche werden vorzugsweise mithin nur einige Carboxylgruppen in dem Additiv benötigt.
Es hat sich gezeigt, dass sich die Lagerstabilität sowie die mechanischen Eigenschaften, insbesondere die Spaltfestigkeit, eines getrockneten oder gehärteten Anwendungsmediums, beispielsweise einer getrockneten Farbe oder Druckfarbe oder eines gehärteten Lackes, verbessern, wenn die Carbonsäuren, vorzugsweise Polycarbonsäuren, als Partialester vorliegen.

Mithin liegt bei dieser bevorzugten Variante noch ein Teil der Carbonsäuren in Form von Carboxyfunktionen vor. Es wird vermutet, dass die Additive mit derartigen Carboxyfunktionen der erfindungsgemäßen Mischung besonders gut an die Metallpigmentoberfläche anzubinden vermögen.

Die erfindungsgemäß zu verwendenden Additive weisen daher bevorzugt Säurezahlen von 5 bis 140, weiter bevorzugt 6 bis 100 mg KOH/g Additiv und besonders bevorzugt von 8 bis 50 KOH/g Additiv auf. Diese Säurezahlen werden bevorzugt nach der DIN 53402 ermittelt.

Gemäß einer weiteren Variante der Erfindung erfolgt die kovalente Kopplung von Carbonsäure und Polyglykolether nicht über eine Veresterung oder eine Amidierung der Carboxyfunktionalitäten der Carbonsäure.

Bei dieser Variante wird beispielsweise das Additiv aus Ausgangsmaterialien, die einerseits eine Carbonsäure mit Hydroxyfunktionen (beispielsweise Weinsäure) und andererseits mit einem Polyglykolether, der eine endständige Epoxidgruppe aufweist, hergestellt. Bei der Carbonsäure werden die freien Carboxyfunktionen durch geeignete Schutzgruppen ihrer Reaktivität mit der Epoxidgruppe enthoben, so dass der Polyglykolether mittels seiner Epoxyfunktion mit der Hydroxyfunktionen der Carbonsäure unter Ausbildung einer α-Hydroxyetherbindung kovalent verknüpft werden.

Gemäß einer weiteren Variante der Erfindung können die Carbonsäure und der Polyglykolether über einen Kohlenwasserstoffrest miteinander gekoppelt sein. Dieser Kohlenwasserstoffrest kann gesättigt oder ungesättigt sein und umfaßt vorzugsweise 2 bis 100 C-Atome. Weiterhin ist bevorzugt, dass der Kohlenwasserstoffrest, 4 bis 50, noch weiter bevorzugt, 6 bis 20, Kohlenstoffatome umfasst. Äußerst bevorzugt weist der Kohlenwasserstoffrest eine Kettenlänge im Bereich von 2 bis 10 Kohlenstoffatome auf. Der Kohlenwasserstoffrest kann Sauerstoffatome enthalten und/oder substituiert sein. Der Kohlenwasserstoffrest ist vorzugsweise geradkettig, kann aber auch verzweigt sein. Um eine kovalente Verbindung von Carbonsäure und Polyglykolether zu bewirken, werden diese mit einem bifunktionellen reaktiven Kohlenwasserstoff umgesetzt. Gemäß einer bevorzugten Variante werden Diglycidylverbindungen, vorzugsweise Diglycidylether verwendet.

Bei bevorzugten Ausführungsformen besitzen die Additive ein gewisses Verhältnis von ihren hydrophilen Polyetherresten und den hydrophoben Kohlenwasserstoffgerüsten der Carbonsäure bzw. der Polycarbonsäuren.
Hierbei ist das Verhältnis der Länge der Polyethereinheiten y+z+k (Polymerisationsgrad) zur Zahl der Kohlenstoffatome der Polycarbonsäure bevorzugt 0,1 bis 4,0, weiter bevorzugt 0,15 bis 3,0, besonders bevorzugt 0,2 bis 2,0 und ganz besonders bevorzugt 0,25 bis 1,0.

Unterhalb eines Verhältnisses von 0,1 kann es sein, dass die Additive keine erfindungsgemäßen Vorteile im Vergleich zu Fettsäuren bewirken. Oberhalb von einem Verhältnis von 4,0 kann es ebenfalls sein, dass keine Vorteile mehr beobachtbar sind. Die Additive tendieren in diesem Fall dazu, nicht mehr fest an die Metalleffektpigmentoberfläche gebunden zu sein.

Gemäß einer weiteren Variante können die Carbonsäuren auch teilweise mit monofunktionellen Alkoholen verestert sein. Der Veresterungsgrad der monofunktionellen Alkohole beträgt bevorzugt 0 bis 50 % der vorhandenen Carbonsäurefunktionen.

Die monofunktionellen Alkohole enthalten einen Kohlenwasserstoffrest mit 1 bis 20 C-Atomen. Der Kohlenwasserstoffrest kann geradkettig oder verzweigt sein und gesättigt oder ungesättigt sein.

Beispiele geeigneter Alkohole sind: Isopropanol, Butanol, t-Butanol, Amylalkohol, Isoamylalkohohl n-Hexanol, 2-Ethylhexan, Myristylalkohol, n-Octanol, Isooctanol, Isodecanol, Caprylalkohol, Laurylalkohol, Stearylakohol, Tridecylalkohol, Hexadecylakohol sowie Mischungen dieser Alkohole.

Vorzugsweise liegt das mittlere Molekulargewicht des erfindungsgemäß auf den Metalleffektpigmenten zu verwendenden Additivs in einem Bereich von 200 bis 20.000 g/mol, weiter bevorzugt von 300 bis 10.000 g/mol. Als sehr geeignet hat sich ein mittlerer Molekulargewichtsbereich von 500 bis 8.000 g/mol erwiesen und besonders bevorzugt ist ein Molekularbereich von 1.000 bis 4.000 g/mol.

Gemäß einer weiteren Variante der Erfindung liegt die Carbonsäure teilweise oder vollständig als Carbonsäuresalz vor.

Die Carbonsäuresalze können als Kationen Alkali- und/oder Erdalkalikationen enthalten. Bevorzugte Die Kationen der Carbonsäuresalze sind dabei bevorzugt Li⁺, Na⁺, K⁺, Mg²⁺ und/oder Ca²⁺ sowie deren Mischungen.

Vorzugsweise ist das Carbonsäuresalz ein Salz aus dem Carboxylat der Carbonsäure und einem oder mehreren Metallkation(en), wobei die Metallkationen vorzugsweise aus den Metallen ausgewählt werden, die in dem Metallkern des Metalleffektpigmentes enthalten sind.

Es hat sich als vorteilhaft herausgestellt, bei Verwendung von Carbonsäuresalzen, beispielsweise Metallseifen, solche auszuwählen, deren Kation(en) mit dem Metall bzw. den Metallen des Metalleffektpigmentes übereinstimmen, um in ein Anwendungsmedium nicht unnötig zusätzliche Ionen einzubringen.

Gemäß einer bevorzugten Variante werden das oder die Metallkation(en) des Carbonsäuresalzes aus der Gruppe der ein-, zwei- und/oder dreiwertigen Metallkationen ausgewählt werden.

Bevorzugte Kationen sind mithin Al³⁺, Fe³⁺, Fe²⁺, Cu²⁺ und/oder Zn²⁺.

Diese Carbonsäuremetallsalze können auch in-situ gebildet werden aus den Carbonsäuren, die auf den Metallpigmenten aufgebracht sind. Die Carbonsäuren können mit der Zeit mit dem Metallpigment unter Bildung der Metallseife reagieren. Dieses Verhalten ist auch von den standardmäßig als Schmiermittel verwendeten Fettsäuren, wie beispielsweise Stearinsäure oder Ölsäure, bekannt.

Gemäß einer bevorzugten Ausführungsform werden die Additive als Schmiermittel zur Vermahlung der Metallpigmente verwendet. Gemäß einer weiter bevorzugten Ausführungsform werden diesem Schmiermittel keine weiteren Zusätze an gesättigten oder ungesättigten Fettsäuren wie Stearin- oder Ölsäuren zugesetzt. Es hat sich gezeigt, dass die vorteilhaften Wirkungen der erfindungsgemäßen Schmiermittel durch die Zugabe von herkömmlichen Fettsäuren während der Vermahlung eher beeinträchtigt werden.

Gemäß einer weiteren Ausführungsform werden die Additive nicht während der Vermahlung zugesetzt, sondern erst nach der Vermahlung. Hier beruht die Wirkung dieser Additive vor allem darauf, dass es als ausgezeichnetes Dispergierhilfsmittel dient. Dabei kann das Metallpigment sowohl in lösemittelhaltigen als auch in wässrigen Anwendungssystemen hervorragend eingearbeitet werden.
Die Additive werden bevorzugt dem Metallpigmentfilterkuchen oder einer Metalleffektpigmentpaste oder einem -pulver zugesetzt.
Insbesondere weisen derartig behandelte Metalleffektpigmente eine deutlich verbesserte Lagerstabilität auf.

Gemäß einer vorteilhaften Weiterbildung der Erfindung liegen die Metalleffektpigmente in kompaktierter Form, vorzugsweise als Granulat, Pellets, Tabletten, Briketts, Würstchen oder als Paste, vor.

Die vorgenannten Darreichungsformen ermöglichen eine staubarme, vorzugsweise staubfreie, Handhabung der erfindungsgemäßen Metalleffektpigmente. Die Metalleffektpigmente können einfach transportiert, dosiert und verarbeitet werden, ohne dass eine Gefahr für Mensch und Umwelt besteht.

Vorzugsweise weisen die Metalleffektpigmente in kompaktierter eine Restfeuchte von bis zu etwa 15 Gew.-%, weiter bevorzugt von etwa 0,1 bis etwa 10 Gew.-%, noch weiter bevorzugt von etwa 0,2 bis etwa 8 Gew.-%, auf, wobei sich die Angabe Gew.-% auf das Gesamtgewicht der kompaktierten Metalleffektpigmentpräparation bezieht.

Ein erfindungsgemäßes Verfahren zur Herstellung der erfindungsgemäßen Metalleffektpigmente umfaßt die folgenden Schritte:
a) Vermahlen von Metallpartikeln zu Metalleffektpigmenten in Gegenwart eines Additivs, das wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind, und Mahlkörpern sowie optional einer Flüssigphase
b) Abtrennen der in Schritt a) erhaltenen mit dem Additiv versehenen Metalleffektpigmente von den Mahlkörpern und optional der Flüssigphase,
c) optional Kompaktieren der in Schritt b) abgetrennten mit dem Additiv versehenen Metalleffektpigmente.

Sämtliche Ausführungen, die in bezug auf die erfindungsgemäßen Metalleffektpigmente und das zu verwendende Additiv vorstehend gemacht wurden, gelten entsprechend im Hinblick auf das Verfahren.

Als Metallpartikel können Metallgrieß, Folienreste oder auch bereits vorverformte Metallplättchen verwendet werden. Der Metallgrieß kann eine spratzige oder eine weitgehend runde Form aufweisen. Im Fall der Herstellung von Aluminium- oder Eisenpigmenten ist eine weitgehend runde Metallgrießform bevorzugt.

Das Verformen des Metallgrießes kann als Trockenvermahlung oder als Naßvermahlung durchgeführt werden. Vorzugsweise erfolgt das Verformen des Metallgrießes als Naßvermahlung.

Als Lösungsmittel können organische Lösemittel, Lösemittelgemische, beispielsweise aus organischem Lösemittel und Wasser, oder wässerige Lösemittel verwendet werden. Bei Verwendung von wässerigem Lösemittel werden vorzugsweise weitere Korrosionsinhibitoren zugesetzt.

Bevorzugt erfolgt die Naßvermahlung jedoch in Gegenwart organischer Lösemittel. Als organische Lösemittel werden vorzugsweise Solventnaphta, Naphtha, Testbenzin, Ester, Ether, Ketone, Alkohole oder Glykole oder Mischungen hiervon verwendet.

Üblicherweise können, wenn sich dies als erforderlich erweist, die Metalleffektpigmente nach der Vermahlung umgenetzt werden. Dies bedeutet, dass sie unter vermindertem Druck und erhöhten Temperaturen von ihrem Lösemittel weitgehend befreit werden und anschließend mit dem für die jeweilige Endanwendung kompatiblen (und vom Kunden gewünschten) Lösemittel wieder angepastet werden.

Stellt man sehr dünne Metalleffektpigmente (mittlere Dicke < 100 nm und besonders ≤ 50 nm) her, so kann es bei dem Umnetzungsschritt jedoch aufgrund der sehr hohen spezifischen Oberflächen der Pigmente zu unerwünschten Agglomerationen der Metallpigmente kommen. In diesem Fall sollte bevorzugt in Lösemitteln vermahlen werden, die kompatibel zu der später geplanten Anmeldung sind.

So sind beispielsweise für eine Anwendung in einer Tiefdruckfarbe Lösemittel wie Ethylacetat, n-Propylacetat oder iso-Propylacetat bevorzugt.

Gemäß einer bevorzugten Variante wird Schritt a) in einer Kugelmühle in Gegenwart von Mahlkörpern, vorzugsweise von sphärischen Mahlkörpern, durchgeführt.

Als sphärische Mahlkörper, vorzugsweise Kugeln, werden bevorzugt Glaskugeln, Stahlkugeln und/oder Keramikkugeln verwendet. Als Keramikkugeln werden vorzugsweise Kugeln aus Korund oder Zirkoniumoxid verwendet.

Der mittlere Kugeldurchmesser liegt vorzugsweise bei 0,3 bis zu 5,0 mm, weiter bevorzugt bei 0,5 bis 4,5 mm und besonders bevorzugt bei 0,6 bis 2 mm.

Die zur Nassvermahlung des Kupfer- oder Messinggrießes eingesetzten Mahlkörper weisen vorzugsweise ein Einzelgewicht von 85 µg bis 515 mg auf.

Gemäß einer bevorzugten Weiterbildung der Erfindung weisen die Mahlkörper ein Einzelgewicht von 0,8 bis 180 mg auf.

Im Falle von Stahlkugeln liegt das mittlere Einzelgewicht vorzugsweise in einem Bereich von 1 bis 180 mg, vorzugsweise von 1,2 bis 150 mg, weiter bevorzugt von 2,0 bis 120 mg. Im Falle von Glaskugeln liegt das mittlere Einzelgewicht in einem Bereich von 1,0 bis 12,5 mg.

Während der Verformungsvermahlung liegt die Temperatur vorzugsweise in einem Bereich von 10°C bis 70°C, weiter bevorzugt von 25°C bis 45°C. Die Mahldauer liegt dabei vorzugsweise in einem Bereich von 2 bis 120 h, vorzugsweise von 5 bis 100 h, noch weiter bevorzugt von 8 bis 80 h.

Für den bevorzugten Fall der Herstellung sehr dünner Metalleffektpigmente (< 100 nm mittlere Dicke) müssen sehr lange Mahldauern veranschlagt werden. In diesem Fall müssen die Pigmente vorsichtig ausgeformt werden. Bevorzugte Mahldauern für Metalleffektpigmente sind mindestens 15 h, weiter bevorzugt mindestens 20 h. Diese Zeiten verstehen sich als Gesamtmahldauerzeiten.
Wenn die Mahlung in zwei oder mehr verschiedenen Schritten durchgeführt wird, so sind entsprechend die Mahldauern der einzelnen Schritte zu addieren.

Bei einem weiteren bevorzugten erfindungsgemäßen Verfahren wird das Additiv nicht als Schmiermittel zur Vermahlung der Metallpigmente eingesetzt. Das Verfahren umfasst dabei folgende Schritte:
a) Vermahlen eines Metallgrießes oder Metallfolienabfällen in Gegenwart von Mahlkörpern und optional Lösemittel mit einem Schmiermittel zu plättchenförmigen Metalleffektpigmenten,
b) Trennen der plättchenförmigen Metalleffektpigmente von den Mahlkörpern und optional vom Hauptteil des Lösemittels,
c) Zugabe und Vermengen des erfindungsgemäß zu verwendenden Additivs zu dem Metallpigmentpulver oder optional Filterkuchen aus Schritt b).

Die Wahl der Lösemittel oder der Mahlkörper (Mahlkugeln) ist dabei die gleiche wie oben beschrieben.

Derartige erfindungsgemäße Mischungen aus Metalleffektpigment und Additiv zeichnen sich durch eine besonders gute Dispergierung der Metalleffektpigmente in einem Anwendungsmedium aus. Durch die Behandlung mit Additiv werden die Metalleffektpigmente weitgehend nicht agglomeriert. Die Metalleffektpigmente zeichnen sich durch eine sehr hohe Lagerstabilität sowie eine leichte Verarbeitbarkeit in Anwendungsmedien wie Druckfarben oder Lacken aus. So kann insbesondere der bei Verwendung von Metalleffektpigmenten in Lacken übliche Aufschlußschritt, bei dem das Metalleffektpigment zusammen mit Lösemittel (in der Regel Butylglycol) sowie ggf. Netzmitteln vordispergiert wird, weitgehend entfallen bzw. lässt sich ohne Netzmittel sehr gut durchführen. Das Metalleffektpigment ist bereits durch das Additiv, welches als Dispergiermittel wirkt, ausgezeichnet aufzuschließen.

Die hierbei verwendete Menge an Additiv hängt vom Einsatzzweck und vor allem der spezifischen Oberfläche des Metalleffektpigmentes ab.

Weiterhin ist bevorzugt, dass das Kompaktieren mittels Granulieren, Pelletieren, Tablettieren, Brikettieren, Filtrieren, Abpressen und/oder Extrudieren durchgeführt wird.

Das Granulieren kann beispielsweise durch Sprühgranulieren erfolgen. Zum Pelletieren werden vorzugsweise Pelletierteller verwendet. Tablettieren und Brikettieren wird vorzugsweise durch Pressen in entsprechende Formen durchgeführt. Die Würstchenform wird vorzugsweise durch Extrudieren der Metalleffektpigmente hergestellt.

Das erfindungsgemäße Metalleffektpigment mit Additiv nach einem der Ansprüche 1 bis 12 werden vorzugsweise zur Herstellung von Beschichtungszusammensetzungen, insbesondere Lacken, Coatings, Druckfarben, Kunststoffen oder kosmetischen Formulierungen verwendet.

Mithin betrifft die vorliegende Erfindung auch eine Beschichtungszusammensetzung, wie beispielsweise Lacke, Coatings, Druckfarben, Kunststoffe oder kosmetischen Formulierungen, die das erfindungsgemäße Metalleffektpigment mit Additiv nach einem der Ansprüche 1 bis 12 enthält.

Bei einer bevorzugten Ausführungsform betrifft die vorliegende Erfindung eine Druckfarbe, die eine erfindungsgemäße Mischung aus Additiv und Metalleffektpigment enthält. Besonders bevorzugt ist hierbei, dass das Additiv als Schmiermittel bei der Herstellung des Metalleffektpigments durch Vermahlung, vorzugsweise durch Naßvermahlung, eingesetzt wird.

Die Druckfarbe ist bevorzugt eine Flüssig-Druckfarbe wie eine Tief-, Flexo- oder Siebdruckfarbe. Selbstverständlich kann es sich jedoch auch um eine Offsetdruckfarbe oder um eine Digitaldruckfarbe handeln.

Im Fall von Digitaldruckfarbe sind besonders Ink-jet-Druckfarben bevorzugt. In diesem Fall können nur sehr feine Metalleffektpigmente verwendet werden. Diese feinen Metalleffektpigmente weisen eine mittlere Größe d₅₀ von 0,65 bis 10 µm, bevorzugt von 0,7 bis 8 µm und besonders bevorzugt von 0,8 bis 6 µm auf. Derart feine Metalleffektpigmente sind hier nötig, da es andernfalls zu einer Verstopfung der Zuleitungen bzw. der Einspritzdüsen kommt.

Die erfindungsgemäßen Tief-, Flexo- oder Siebdruckfarben enthalten Lösemittel bzw. Lösemittelgemische. Diese dienen u.a. zum Lösen der Bindemittel, aber auch zur Einstellung wichtiger Anwendungseigenschaften der Druckfarben, wie beispielsweise der Viskosität oder der Trocknungsgeschwindigkeit.

Für Flüssig-Druckfarben wie Flexo- und Tiefdruckfarben eingesetzte Lösemittel umfassen insbesondere niedrig siedende Lösemittel. Der Siedepunkt beträgt im Regelfalle nicht mehr als 140°C. Höher siedende Lösemittel werden nur in kleineren Mengen zur Einstellung der Trocknungsgeschwindigkeit eingesetzt. Siebdruckfarben sind ähnlich formuliert wie Flexo- oder Tiefdruckfarben, sie sind lediglich etwas höher viskos eingestellt und weisen üblicherweise Lösemittel mit etwas höheren Siedepunkten auf. Beispiele geeigneter Lösemittel für Flüssigdruckfarben umfassen Ethanol, 1 -Propanol oder 2-Propanol, substituierte Alkohole wie beispielsweise Ethoxypropanol oder Ester wie beispielsweise Ethylacetat, Isopropylacetat, n-Propyl- oder n-Butylacetat. Es können selbstverständlich auch Gemische verschiedener Lösemittel eingesetzt werden. Beispielsweise kann es sich um ein Gemisch aus Ethanol und Estern wie Ethylacetat oder Propylacetat handeln. Für das Drucken mit Flexodruckplatten ist es regelmäßig empfehlenswert, dass der Anteil der Ester am Gesamtlösemittel ca. 20 - 25 Gew.-% nicht überschreitet. Als Lösemittel für Flüssigdruckfarben sind bevorzugt auch Wasser oder überwiegend wässrige Lösemittelgemische einsetzbar.

Je nach Art der Druckfarbe werden üblicherweise 10 bis 60 Gew.-% Lösemittel bezüglich der Summe aller Bestandteile eingesetzt. Im Fall der erfindungsgemäßen Druckfarben erweist sich jedoch ein Bereich von 60 - 80 Gew.-% Lösemittel als besonders vorteilhaft.

Strahlungshärtbare Druckfarben enthalten im Allgemeinen nicht die oben genannten Lösemittel, sondern Reaktivverdünner. Reaktivverdünner erfüllen typischerweise eine Doppelfunktion. Einerseits dienen sie zum Vernetzen bzw. Härten der Druckfarbe, andererseits dienen sie aber auch wie konventionelle Lösemittel (DE 20 2004 005 921 UI 2004.07.1 zum Einstellen der Viskosität. Beispiele umfassen Beispiele umfassen Butylacrylat, (2-Ethylhexyl)acrylat, sowie insbesondere mehrfunktionelle Acrylate wie 1,4-Butandioldi(meth)acrylat, 1,6-Hexandioldi(meth)acrylat oder Trimethylolpropantri(meth)acrylat.

Als Bindemittel für die erfindungsgemäßen Metallic-Druckfarben können prinzipiell die für Flüssig-Druckfarben üblichen Bindemittel eingesetzt werden. Je nach dem gewünschten Anwendungszweck und den gewünschten Eigenschaften trifft der Fachmann eine geeignete Auswahl. Beispiele geeigneter Bindemittel umfassen Polyester, Polyamide, PVC-Copolymerisate, aliphatische und aromatische Ketonharze, Melamin-Harnstoff-Harze, Melamin-Formaldehyd-Harze, Maleinate, Kolophoniumderivate, Casein bzw. Casein-Derivate, Ethylcellulose, Nitrocellulose oder aromatische bzw. aliphatische Polyurethane. Es können auch Polymere oder Copolymere von Vinylacetat, Vinylalkohol, Acrylaten, Methacrylaten, Vinylpyrrolidon oder Vinylacetalen eingesetzt werden. Von besonderem Vorteil können funktionelle Gruppen aufweisende hyperverzweigte Polymere, beispielsweise hyperverzweigte Polyurethane, Polyharnstoffe oder Polyesteramide eingesetzt werden, wie in den WO 02/36695 und WO 02/36697 offenbart. Es können selbstverständlich auch Gemische verschiedener polymerer Bindemittel eingesetzt werden, vorausgesetzt, die ausgewählten Bindemittel weisen in Kombination miteinander keine unerwünschten Eigenschaften auf. Die Menge aller Bindemittel beträgt üblicherweise 5 - 40 Gew.-% bzgl. der Summe aller Bestandteile der Druckfarbe.

Besonders bevorzugte Bindemittel umfassen beispielsweise Nitrocellulose, Ethylcellulose, Hydroxyethylcellulose, Acrylate, Polyvinylbutyrale sowie aliphatische und aromatische Polyurethane und Polyharnstoffe, insbesondere hyperverzweigte Polyurethane und Polyharnstoffe sowie Mischungen davon.

Als Bindemittel für wasserverdünnbare Metallic-Druckfarben kommen insbesondere Copolymere auf Basis von (Meth)acrylsäure und/oder deren Estern mit Styrol in Frage. Derartige Bindemittel sind als Lösungen oder Dispersionen für den Einsatz in Druckfarben beispielsweise unter dem Namen Zinpol® (Fa. Worlee) kommerziell erhältlich. Weitere Beispiele umfassen aromatische bzw. aliphatische wässrige Polyurethane, Polyester und auf wässrige Polyamide.

Für pastöse Druckfarben bevorzugte Bindemittel umfassen beispielsweise Kolophoniumharze oder modifizierte Kolophoniumharze. Beispiele für modifizierte Kolophoniumharze umfassen mit Polyolen wie beispielsweise Glycerin oder Pentaerythrit ganz oder teilweise veresterte Kolophoniumharze.

Strahlungshärtbare Druckfarben umfassen Bindemittel, die vernetzbare Gruppen umfassen, wie beispielsweise olefinische Gruppen, Vinylether- oder Epoxidgruppen. Hier liegt die Summe der Bindemittel (inklusive Reaktivverdünner) in der Regel in einem Bereich von 30 - 90 Gew.-% aller Bestandteile der Druckfarbe.

Die erfindungsgemäßen Metallic-Druckfarben können weiterhin einen oder mehrere Hilfsstoffe beziehungsweise Additive umfassen. Beispiele für Additive und Hilfsstoffe sind Füllstoffe wie Calciumcarbonat, Aluminiumoxidhydrat oder Aluminium- bzw. Magnesiumsilikat. Wachse erhöhen die Abriebfestigkeit und dienen der Erhöhung der Gleitfähigkeit. Beispiele sind insbesondere Polyethylenwachse, oxidierte Polyethylenwachse, Petroleumwachse oder Ceresinwachse. Fettsäureamide können zur Erhöhung der Oberflächenglätte eingesetzt werden. Weichmacher dienen der Erhöhung der Elastizität des getrockneten Films. Für strahlungshärtbare Druckfarben wird als Additiv weiterhin mindestens ein Fotoinitiator oder ein Fotoinitiatorsystem eingesetzt. Zum Dispergieren der Effektpigmente können Dispergierhilfsmittel eingesetzt werden. Mittels Fettsäuren kann ein Aufschwimmen der Metalleffektpigmente in der gedruckten Schicht erreicht werden, so dass die Pigmente in an der oberen Grenzfläche der Druckschicht angereichert sind. Hierdurch können vorteilhaft verbesserte Metallic-Effekte erzielt werden. Weiterhin können auch Antiabsetzmittel zugesetzt werden. Derartige Zusätze verhindern die Sedimentation der Effektpigmente. Beispiele umfassen Kieselsäure, CelluloseDerivate oder auch Wachse. Bei den erfindungsgemäßen Metalleffektpigmenten kann jedoch vorteilhaft von der Verwendung von Absetzmitteln abgesehen oder der Anteil an Absetzmitteln verringert werden.

Zur Formulierung der besonders bevorzugten dünnflüssigen Flexo-, Tief- oder Siebdruckfarben ist der Zusatz von Antiabsetzmitteln meist empfehlenswert, wenngleich nicht immer unbedingt erforderlich. Die Gesamtmenge aller Additive und Hilfsstoffe sollte üblicherweise 20 Gew.-% bezüglich der Summe aller Bestandteile der Druckfarbe nicht übersteigen und beträgt bevorzugt 0,1 -10 Gew.-%.

Die Herstellung der erfindungsgemäßen Metallic-Druckfarben kann in prinzipiell bekannter Art und Weise durch intensives Vermischen bzw. Dispergieren der Bestandteile in üblichen Apparaturen wie beispielsweise Dissolvern oder Rührwerken erfolgen. Der Fachmann wird beim Einsatz von Dissolvern darauf achten, dass der Energieeintrag nicht zu hoch ist, um ein Beschädigen der erfindungsgemäßen Metalleffektpigmente zu vermeiden. Umgekehrt muss er natürlich so hoch sein, um ein ordnungsgemäßes Dispergieren der Pigmente zu ermöglichen. Falls neben den erfindungsgemäßen Metalleffektpigmenten noch übliche Farbpigmente eingesetzt werden, kann es empfehlenswert sein, diese in einem Teil oder in der Gesamtmenge des Lösemittels, Bindemittels sowie gegebenenfalls der Hilfsstoffe der Metallic-Druckfarbe vorzudispergieren, und die erfindungsgemäßen Metalleffektpigmente erst später zuzugeben. Auf diese Art und Weise wird eine besonders gute Dispergierung der zusätzlichen Pigmente erreicht, ohne die Metalleffektpigmente durch zu starke Dispergierung zu schädigen. Anstelle der Pigmente können auch vordispergierte Pigmentkonzentrate zugegeben werden. Ganz besonders elegant kann hierbei auch eine handelsübliche Druckfarbe in geringen Mengen eingesetzt werden, vorausgesetzt, die zugesetzte Druckfarbe ist mit der Rezeptur der Metallic-Druckfarbe verträglich und verschlechtert nicht deren Eigenschaften.

Die nachfolgenden Beispiele erläutern die Erfindung näher, ohne sie jedoch zu beschränken.

### A Beispiele Aluminiumeffektpigmente

### Beispiel 1a:

50 g Pripol 1009 (hydrierte C36-Dimersäure von Unichema) und 89 g MPEG 750 (Methoxypolyethylenglycol) wurden in ein Glasreaktionsgefäß eingewogen und unter N₂-Schutzgas und Rühren auf 80 °C erwärmt. Anschließend wurde 0,8 g p-Toluolsulfonsäure (Katalysator) hinzugegeben und auf 180 °C aufgeheizt.

Entstehendes Reaktionswasser wurde über einen Wasserabscheider abgeschieden. Anhand der Säurezahl wurde der Fortschritt der Reaktion kontrolliert. Die Säurezahl wurde gemäß der DIN 53402 bestimmt. Die Reaktion wurde nach Erreichen einer Säurezahl von etwa 24 mg KOH/g Additiv gestoppt. Dies entspricht einem Veresterungsgrad von ca. 67 %. Das mittlere Molekulargewicht des entstandenen Esters betrug ca. 1.750 g/ mol, wobei das Verhältnis der Ethereinheiten zu C-Atomen: ca. 0,3 betrug.

### Beispiel 1b:

100 g verdüster Aluminiumgrieß mit den Korngrößenverteilungsparametern (mittlere Partikelgröße d_{50, Grieß} = 2,2 µm, d_{10, Grieß} -Wert 1,1 µm, d_{90, Grieß}-Wert 3,6 µm) und 440 g Isopropylacetat sowie 8 g des Additivs aus Beispiel 1a wurden in eine Topfmühle (Länge: 32 cm, Breite: 19 cm) gefüllt und die Mühle geschlossen. Anschließend wurde 12 h lang mit 4,5 kg Stahlkugeln (Durchmesser: 1,8 mm) mit 50 U/min vermahlen. Anschließend wurde in einer zweiten Mahlstufe 13 h lang bei 24 U/min vermahlen. Das aus der Mühle entleerte Vermahlungsprodukt wurde mit Isopropylacetat ausgewaschen und mittels einer Siebung (24 µm) von den Mahlkugeln getrennt. Das Siebgut wurde mittels einer Nutsche weitgehend von Isopropylacetat befreit und anschließend wiederum mit Isopropylacetat in einem Labormischer angepastet (ca. 65 Gew.-% Feststoffanteil).

### Beispiel 2:

Vermahlungsrezeptur wie Beispiel 1 b, jedoch wurde als Schmiermittel 8 g des kommerziell erhältlichen Fettsäurepolyglykolester P 4100 (Fa. Byk, Wesel, Deutschland) eingesetzt,

### Beispiel 3:

Vermahlungsrezeptur wie in Beispiel 1 b, jedoch wurde ein Aluminiumgrieß mit einer mittleren Partikelgröße d₅₀, _{Grieß} = 1,7 µm, d_{10, Grieß} -Wert 0,4 µm, d₉₀, _{Grieß} -Wert 2,3 µm eingesetzt.

### Beispiel 4:

Vermahlung wie bei Beispiel 3, jedoch wurde als Schmiermittel 8 g des kommerziell erhältlichen Dispergieradditivs P4100 (Fa. Byk, Wesel, Deutschland) eingesetzt.

### Vergleichsbeispiel 5:

Vermahlung wie in Beispiel 1b, jedoch wurde als Schmiermittel 3 g einer gewöhnlichen Mischung aus Stearin- und Ölsäure eingesetzt.

### Vergleichsbeispiel 6:

Vermahlung wie in Beispiel 3, jedoch wurde als Schmiermittel 3 g einer gewöhnlichen Mischung aus Stearin- und Ölsäure eingesetzt.

### Vergleichsbeispiel 7:

Kommerziell erhältliches Aluminiumpigment (Platindollarpigment) Platinvario 85001 (Fa. Eckart, Deutschland).
Hier wurde als Schmiermittel eine gewöhnliche Mischung aus Stearin- und Ölsäure eingesetzt.

### Vergleichsbeispiel 8: VP 55000

Kommerziell erhältliches non-leafing Silberdollarpigment VP 55000 (Fa. Eckart, Deutschland). Hier wurde als Schmiermittel eine gewöhnliche Mischung aus Stearin- und Ölsäure eingesetzt.

Die Aluminiumeffektpigmente wurde mittels üblicher Laserbeugungsmethoden (Gerät: Cilas 1064, Fa. Cilas, Frankreich) hinsichtlich seiner Größenverteilung bestimmt. Mittels der in der WO 2004/087816 A2 beschriebenen Methode wurde die mittlere Dicke h₅₀ anhand von REM-Zählungen ermittelt. Die Ergebnisse dieser Pigmentcharakterisierung zeigt Tabelle 1.

**Tab. 1: Physikalische Charakterisierung der Pigmente**

| **Probe** | **Schmier mittel** | **Partikelgrößenverteilung** | | | **Mittlere Dicke (REM)** h₅₀/nm |
|---|---|---|---|---|---|
| | | d₁₀/µm | d₅₀/µm | d₉₀/µm | |
| Beispiel 1b | Beispiel 1a | 4,3 | 9,4 | 15,1 | ca. 50 |
| Beispiel 2 | P 4100 | 4,1 | 9,2 | 14,7 | |
| Beispiel 3 | Beispiel 1a | 4,8 | 11,1 | 18,7 | ca. 50 |
| Beispiel 4 | P 4100 | 4,4 | 10,6 | 18,3 | |
| Vergleichsbeispiel 5 | Mischung Stearin-/Ölsäure | 4,3 | 9,1 | 14,6 | ca. 55 |
| Vergleichsbeispiel 6 | " | 4 | 9,7 | 17,1 | ca. 65 |
| Vergleichsbeispiel 7 | " | 5,8 | 10,2 | 15,6 | 60 |
| Vergleichsbeispiel 8 | " | 4,7 | 9,1 | 14,6 | --- |

### Ergebnisse der optischen Abprüfung:

Die Pigmente der erfindungsgemäßen Beispiele und der Vergleichsbeispiele wurden in folgenden Prüfsystemen für Lackierungen (Nitrolack) und Drucke abgeprüft.

Hierbei wurden folgende Systeme verwendet:
a) Tiefdruckfarbe auf Basis kommerziell erhältlicher Nitrocellulose H 33 mit einer Pigmentierungshöhe von 11,0 Gew.-% und einem Ethanol/Ethylacetat Lösemittelgemisch.
b) Tiefdruckfarbe auf Basis kommerziell erhältlicher Acrylatbindemittel mit einer Pigmentierungshöhe von 10,8 Gew.-%, und einem Ethanol/Ethylacetat Lösemittelgemisch.
c) Rückseitenapplikationen (Spiegellack) auf Basis eines kommerziell erhältlichen Polyvinylbutyralbindemittels mit einer Pigmentierungshöhe von 9,8 Gew.-% und einem Ethanol/Ethylacetat Lösemittelgemisch.
   Die Rückseitenapplikationen in Tab. 2 wurden mit Hilfe einer Tiefdruckfarbe auf Basis eines kommerziell erhältlichen Polyvinylbutyrals durch Bedrucken einer MELINEX 400 Folie (PET-Folie, 50 µm) mittels einer Rakel mit einer Rillentiefe von 24 µm erstellt.
d) Nitrolackabzüge: Verwendet wurde der kommerziell erhältliche Nitrolack Dr. Renger Erco Bronzemischlack 2615e (Fa. Morton) bei einer Pigmentierungshöhe von 7,1 Gew.-% und einer Rakeltiefe von 24 µm.

### Glanz:

Die Glanzwerte wurden jeweils mit dem Messgerät Micro-Tri-Gloss, (Fa. Byk-Gardner, Geretsried, Deutschland) bei einem Meßwinkel von 60 erhalten. Das Gerät wurde hier mittels Dunkelkalibrierung sowie einer schwarzen Spiegelglasplatte mit Werten von 95,5 für 60° kalibriert.

### Spaltfestigkeit:

Zur Überprüfung der Spaltfestigkeit zwischen Pigment und Bindemittel der Beispiele und Vergleichsbeispiele wurden Rakelabzüge aus den oben erwähnten Lacken bzw. Farben angefertigt. Auf diese wurde nach vollständigem Aushärten der Farbe bzw. der Lackschicht ein Klebestreifen fest und ohne Blasen auf die Oberfläche geklebt. Anschließend wurde dieser Klebestreifen wieder abgezogen, so dass der Untergrund (z. B. Papier) nicht beschädigt wurde. Die Spaltfestigkeit wurde anhand eines Notensystems visuell beurteilt. Eine schlechte Spaltfestigkeit spiegelt sich in einem entsprechend starken Ausriss aus dem Druck bzw. der Lackierung wieder.

### Deckung / Transferverhalten:

Das Deckungs- bzw. Transferverhalten der Beispiele und Vergleichsbeispiele wurde mittels Andrucken ermittelt.
Unter Transferverhalten wird die Übertragung von Metallpigment von der Druckplatte (bzw. -näpfchen) auf den bedruckten Gegenstand verstanden. Das Transferverhalten wurde anhand eines Notensystems visuell beurteilt. Andrucke mit schlechtem Transferverhalten sind anwendungstechnisch in keiner Weise akzeptabel. Ein schlechtes Transferverhalten bewirkt natürlicherweise auch ein schlechtes Deckungsverhalten, da weniger Pigment in der Applikation vorhanden ist.
- Note 0:: sehr gut
- Note 1:: gut
- Note 2:: befriedigend
- Note 3:: ausreichend
- Note 4:: schlecht
- Note 5:: sehr schlecht

Es zeigte sich, dass die Glanzwerte im Einzelnen entscheidend von den verwendeten Lack- bzw. Druckfarbensystemen abhängen. Ebenso variiert die Zwischenhaftung in Abhängigkeit des Bindemittels. Generell kann jedoch bei den Applikationen mit den erfindungsgemäßen Pigmenten einer gute bis sehr gute Spaltfestigkeit bei relativ hohem Glanz und einem befriedigenden bis sehr guten Deckungs-/Transferverhalten beobachtet werden.
Die erfindungsgemäßen Beispiele weisen in der Kombination dieser Eigenschaften ein deutlich besseres Verhalten auf als die Vergleichsbeispiele.
Im Einzelnen können bei Vergleichsbeispiel 7 auch sehr hohe Glanzwerte in den Tiefdruckfarben beobachtet werden. Jedoch zeigt dieses Pigment eine schlechte Spaltfestigkeit und ein schlechtes Deckungs-Transferverhalten auf.

Das Vergleichsbeispiel 6 weist eine sehr ähnliche Pigmentgrößenverteilung auf wie Beispiel 1 b. Jedoch ist bei den Tiefdruckanwendungen der Glanz geringer und teilweise das Transferverhalten schlecht. In der Spiegelapplikation wird ein sehr hoher Glanz erzielt, jedoch ist die Spaltfestigkeit weniger gut.

In der Nitrolackapplikation tritt im Vergleich zu den Vergleichsbeispielen 5 und 6 keine Verschlechterung des Glanzwertes auf; es kann sogar eine Steigerung um 30 % erreicht werden.

**Tab. 2: Ergebnisse der Abprüfungen Aluminiumpigmente**

| **Probe** | **Prüfung Tiefdruckfarbe a) Nitrocellulose** | | | **Prüfung Tiefdruckfarbe b) Acrylat** | | | **c) Spiegellack-applikation** | | **Nitro-lack** | |
|---|---|---|---|---|---|---|---|---|---|---|
| | **Glanz (60°)** | **Spaltfestigkeit** | **Deckung/ Transfer** | **Glanz (60°)** | **Spaltfestigkeit** | **Deckung/ Transfer** | **Glanz (60°)** | **Spaltfestigkeit** | **Glanz (60°):** | **Spaltfestigkeit:** |
| Beispiel 1b | 127 | gut (1) | sehr gut (1) | 80,3 | sehr gut(0) | gut (2) | 495 | sehr gut (0) | 123 | sehr gut (0) |
| Beispiel 2 | 120 | sehr gut (0) | sehr gut (0) | 75,4 | sehr gut (0) | weniger gut (3) | 494 | schlecht (4) | 91,7 | sehr gut (0) |
| Beispiel 3 | 137 | gut (1) | gut (1) | 78,5 | sehr gut (0) | gut (2) | 465 | weniger gut (3) | 106 | sehr gut (0) |
| Beispiel 4 | 119 | sehr gut (0) | sehr gut (0) | 79,8 | sehr gut (0) | gut (2) | 507 | gut (1) | 98,9 | sehr gut (0) |
| Vergleichsbeispiel 5 | 101 | weniger gut (3) | gut (2) | 70,0 | sehr gut (0) | gut (2) | 466 | gut (2) | 61,7 | sehr gut (0) |
| Vergleichsbeispiel 6 | 116 | gut (2) | schlecht (3) | 75,6 | sehr gut (0) | gut (2) | 522 | weniger gut (3) | 109 | gut (0) |
| Vergleichsbeispiel 7 | 122 | schlecht (5) | schlecht (4) | 82,5 | schlecht (4) | schlecht (4) | 605 | schlecht (4) | 131 | schlecht (5) |
| Vergleichsbeispiel 8 | 88,7 | schlecht (4) | schlecht (3) | 69,2 | weniger gut (3) | schlecht (4) | 357 | schlecht (4) | 60,5 | schlecht (4) |

### Beispiele für erfindungsgemäße Metalleffektpigmente mit Additiv und Vergleichsbeispiele:

Neben der Verbesserung der anwendungstechnischen Eigenschaften von Aluminiumeffektpigmenten durch Verwendung der Additive als Schmiermittel während der Vermahlung kann das Additiv auch zur nachträglichen Stabilisierung von Pigment-Filterkuchen verwendet werden.

### Vergleichsbeispiel 9:

Kommerziell erhältliches Silberdollarpigment MEX 2192 (Fa. Eckart).
Dieses Pigment wird mit einem Gemisch aus Stearin- und Ölsäure vermahlen.

### Beispiel 10:

Kommerziell erhältliches Silberdollarpigment MEX 2192 wird nach der Vermahlung im Filterkuchen mit 2 Gew.-% Additivs gemäß Beispiel 1 a vermengt, in einem Mischer homogenisiert und anschließend zu einer Metalleffektpigmentpaste weiterverarbeitet.

Bekanntermaßen neigen Metallpigmentpasten bei längerer Lagerung zum Bilden von Agglomeraten, die sich auch durch intensivere Dispergierung nicht mehr aufschließen lassen. Als allgemein anerkannter Test für dieses Verhalten werden Proben in verschlossenen Druckdeckeldosen bei 50 °C eingelagert und in regelmäßigen Abständen auf Agglomerate (Rakelabzüge auf Kontrastkarton) untersucht. Die erhöhten Temperaturen simulieren eine wesentlich längere Lagerungszeit unter Normalbedingungen.

**Tab. 3: Ergebnisse der Lagerung bei 50 °C in Tagen:**

| Probe | Dauer Lagerstabilität |
|---|---|
| Vergleichsbeispiel 9 (Mex 2192) | 28 d |
| Beispiel 10 | > 140 d |

Um die für die Druckanwendung relevanten Parameter Deckung und Glanz zu überprüfen, wurde das erfindungsgemäße Metalleffektpigment nach der oben beschriebenen Lagerung in einer Tiefdruckfarbe auf Basis kommerziell erhältlicher Nitrobindemittel (LQ 2903) auf Folie appliziert. Die Glanzwerte und die Deckung wurden in der oben beschriebenen Weise ermittelt.

### Beispiel 11:

Kommerziell erhältliches Silberdollarpigment IL Reflexal VP-62617/G (Fa. Eckart) wird nach der Vermahlung im Filterkuchen mit 2 Gew.-% eines Additivs gemäß Beispiel 1 a vermengt und anschließend zu einer Metalleffektpigmentpaste weiterverarbeitet.

### Vergleichsbeispiel 12:

Kommerziell erhältliches Silberdollarpigment IL Reflexal VP-62617/G (Fa. Eckart).

### Beispiel 13:

Kommerziell erhältliches Silberdollarpigment AF Reflexal VP-58187/G wird nach der Vermahlung im Filterkuchen mit 2 Gew.-% eines Additivs gemäß Beispiel 1 a vermengt und anschließend zu einer Metalleffektpigmentpaste weiterverarbeitet.

### Vergleichsbeispiel 14:

Kommerziell erhältliches Silberdollarpigment AF Reflexal VP-58187/G (Fa. Eckart).

**Tab. 4: Ergebnisse der optischen Abprüfung nach 6 Wochen Lagerung bei 50 °C**

| | Deckung | | Glanz | |
|---|---|---|---|---|
| **Probe** | frisch | nach 6 Wochen | frisch | nach 6 Wochen |
| Beispiel 11 | mittel (3) | mittel (3) | 255 | 223 |
| Vergleichsbeispiel 12 | mittel (3) | geringer (1) | 247 | 200 |
| Beispiel 13 | mittel (3) | mittel (3) | 154 | 155 |
| Vergleichsbeispiel 14 | mittel (3) | mittel (3) | 150 | 141 |

Die Metalleffektpigmente gemäß Beispiel 11 weisen nach der Lagerung eine vergleichsweise gleiche Deckung und einen etwas geringeren Glanz in der Anwendung auf. Die Metalleffektpigmente gemäß Vergleichsbeispiel 12 weisen ebenfall eine Glanzerniedrigung auf. Der Glanz ist jedoch generell geringer als bei Beispiel 1. Dagegen nimmt die Deckfähigkeit bei Vergleichsbeispiel 12 ab, was auf beginnende Agglomerate schließen lässt.
Die Metalleffektpigmente gemäß Beispiel 13 weisen praktisch keinerlei Veränderungen hinsichtlich Deckung und Glanz nach der Lagerung auf. Die Metalleffektpigmente gemäß Vergleichsbeispiel 14 dagegen weisen zwar keine Änderung des Deckungsverhaltens, jedoch eine merkliche Glanzeinbuße auf.

### B Beispiele Goldbronzeeffektpigmente

### Beispiel 15:

### a) Verdüsung Metallgrieß:

Zur Herstellung von erfindungsgemäßen Messingpigmenten wurden in einem Induktionsofen 70 Gew.-% Kupfer und 30 Gew.-% Zink eingebracht und aufgeschmolzen. Anschließend wurde die Messingschmelze in einen Induktionsrinnenofen mit Vorherd übergeben. Die im Vorherd bei einer Temperatur von etwa 1050°C flüssig vorliegende Messingschmelze wurde durch eine am Vorherd angebrachte Zerstäubungsdüse/vertikal nach unten verdüst. Zum Zerstäuben der Messingschmelze wurde eine sogenannte geschlossene Düse (close coupled) eingesetzt. Die bei der Verdüsung entstandenen Messingpartikel erstarren und erkalten im Fluge. Die Verdüsung erfolgte unter Zufuhr von Heißluft bei etwa 400 °C. Das zur Verdüsung eingesetzte Heißgas wurde verdichtet, danach in Gaserhitzern erhitzt und anschließend in die zu zerstäubende Messingschmelze eingetragen. Die Abscheidung der Messingpartikel erfolgte mittels Zentrifugalkraft. Der dort abgeschiedene pulverförmige Messinggrieß hatte einen d₅₀ von < 60 µm. Die Gas-Feststoff-Trennung erfolgte in einem Filter. Die weitere Auftrennung dieses Messinggrießes erfolgte weitere Klassierschritte. Daraus resultierte ein pulverförmiger Messingfeinstgrieß ("Messing 70:30 Reichgold"), mit einem d₁₀ von 1,4 µm, einem d₅₀ von 2,4 µm und einem d₉₀ von 4,0 µm sowie einen d₉₈ von 6 µm hergestellt.

### b) Vermahlung:

Zur Nassvermahlung des gemäß Schritt a) hergestellten Messingfeinstgrießes wurden 400 g dieses Metallgrießes in eine Mühle (Länge: 32 cm, Breite: 19 cm) mit 10 kg Chromstahlkugeln (Durchmesser: 3 mm) und 900 g Isopropylacetat sowie 30 g Mahladditiv nach Bsp. 1b eingebracht und bei 80 U/min 30 h lang vermahlen. Das Mahlprodukt wurde durch Spülen mit Lösemittel von den Mahlkugeln getrennt und abfiltriert. Der Filterkuchen wurde anschließend in eine zweite Mühle eingebracht. Die in diese Mühle mit einer Menge von 400 g eingebrachte Messingpaste wurde mit Hilfe von 10 kg Chromstahlkugeln (Durchmesser: 1,3 mm;) mit einer Drehzahl von 60 U/min 30 h lang mit ca. 900 g Isopropylacetat und etwa 25 g Mahladditiv nach Bsp. 1 b vermahlen. Anschließend wurde die Messingpigmentpaste durch Spülen mit Lösemittel von den Mahlkugeln getrennt und danach auf einen Feststoffgehalt von 70 Gew.-% aufkonzentriert.

### Beispiel 16:

Analog Beispiel 15, jedoch wurde bei der Vermahlung anstelle von Isopropylacetat als Lösemittel N-Propylacetat eingesetzt.

### Vergl.-Beispiel 17:

Die Herstellung von kommerziell gehandeltem Goldbronzepigmentpulver für Tief- und Flexodruckfarben ("ROTOFLEX"der Fa. Eckart GmbH) erfolgt im bekannten mehrstufigem Trockenmahlverfahren (Hametag- Verfahren) mit Stearinsäure als Vermahlungshilfsmittel. Als Ausgangsmaterial wurde ein Messinggrieß mit 70 Gew.% Kupfer und 30 Gew.% Zink mit einem mittleren Teilchendurchmesser d₅₀ von 140 µm eingesetzt. Aus den als klassiertes Mahlgut vorliegenden leafing Goldbronzepigmenten mit einem mittleren Teilchendurchmesser d₅₀ = 8µm wurde mittels einer nachfolgenden Oberflächenmodifizierung mit 2,5 Gew.-% Zitronensäure ein Goldbronzepigment mit non leafing Eigenschaften hergestellt.

### Vergl.-Beispiel 18:

Kommerziell erhältliches PVD-Pigment Metalure A (Fa. Eckart GmbH, Deutschland). Dieses Pigment ist in der kommerziell erhältlichen Druckfarbe ("ULTRASTAR" Fa. Eckart) für den Tief- und Flexodruck vorhanden.

Diese Druckfarbe enthält weiterhin einen gelben und einen orangen Tonerfarbstoff, um einen goldfarbenen Eindruck hervorzurufen.

Zur Bestimmung der Teilchendicken wurden Proben des Beispiels15 bis 18 mittels eines Feldionen-Rasterelektronenmikroskops charakterisiert.

Für die Bestimmung der Dickenverteilung mittels REM wurden die Proben folgendermaßen präpariert:

Die aus nassvermahlenem Messinggrieß hergestellten, als Paste oder Filterkuchen vorliegenden, plättchenförmigen Messingpigmente, werden mit Aceton gewaschen und anschließend getrocknet.

Ein in der Elektronenmikroskopie übliches Harz, beispielsweise TEMPFIX (Gerhard Neubauer Chemikalien, D-48031 Münster, Deutschland), wird auf einen Probenteller aufgebracht und auf einer Heizplatte bis zum Erweichen erhitzt. Nachfolgend wird der Probenteller von der Heizplatte genommen und das Messingpulver auf das erweichte Harz gestreut. Das Harz wird durch die Abkühlung wieder fest und die aufgestreuten Messingpigmente können - bedingt durch das Wechselspiel zwischen Adhäsion und Schwerkraft - nahezu senkrecht stehend und fixiert auf dem Probenteller präpariert werden. Dadurch sind die Messingpigmente im Elektronenmikroskop seitlich gut zu vermessen. Bei der Vermessung der Dicke wird der azimuthale Winkel des Pigmentes zu einer zur Oberfläche normalen Ebene geschätzt und bei der Dickenauswertung nach der Formel ${h}_{eff} = {h}_{mess} / cos α$
berücksichtigt.

Von den errechneten h_{eff}-Werten wird anhand der relativen Häufigkeiten die Summenverteilungskurve erstellt. Es werden jeweils 50 bis 100 Teilchen ausgezählt.

Aus der nachfolgenden **Tab. 5** ist die physikalische Charakteristik der erfindungsgemäßen Messingpigmente (Beispiel 15) im Vergleich zu kommerziell gehandeltem Goldbronzepigmentpulver (Vergleichsbeispiel 17) der Fa. Eckart und dem PVD-Aluminiumpigment (Vergleichsbeispiel 18) der Fa. Eckart anhand der d₁₀-, d₅₀- und d₉₀-Werte und die Kennwerte h₁₀, h₅₀ und h₉₀ und daraus berechneten Span-Werte der Dickenmessung aus den REM-Untersuchungen zu entnehmen. Die h₁₀, h₅₀ und h₉₀-Werte wurde mit Hilfe der Quantilfunktion aus den Originaldaten der Dickenauszählung berechnet.

Die Längsausdehnung d der Pigmente wurde mit Hilfe eines Lasergranulometers (Cilas 1064, Firma Cilas, Frankreich) bestimmt und als Maß der mittleren Längsausdehnung wie üblich der d₅₀-Wert der Summendurchgangsverteilung in µm gewählt.

**Tab. 5: Physikalische Charakterisierung Goldbronzepigmente**

| | **Größen** | | | **Dicken** | | | | Formfaktor |
|---|---|---|---|---|---|---|---|---|
| | d₁₀ | d₅₀ | d₉₀ | h₁₀ | h₅₀ | h₉₀ | Span Dickenverteilung | |
| **Proben** | [µm] | [µm] | [µm] | [nm] | [nm] | [nm] | [%] | |
| Beispiel 15 | 3,5 | 8,3 | 13 | 20,2 | 26,2 | 35,8 | 0,6 | 317 |
| Beispiel 16 | 2,9 | 8,2 | 12,6 | 13,6 | 18,3 | 25,8 | 0,67 | 448 |
| Vergleichsbeispiel 17 | 3,3 | 8 | 15 | 29,9 | 45,2 | 74,1 | 1 | 177 |
| Vergleichsbeispiel 18 | 3,5 | 10 | 17,5 | 21 | 29 | 38,7 | 0,6 | 476 |

Aus den Werten der **Tab. 5** ist zu entnehmen, dass die erfindungsgemäßen non-leafing Messingpigmente gemäß Beispiel 15 und 16 sowohl eine geringere mittlere Dicke h₅₀ als auch einen geringeren h₉₀-Wert als die stabilisierten leafing Goldbronzepigmente "ROTOFLEX" der Fa. Eckart GmbH, D-90763 Fürth gemäß Vergleichsbeispiel 17 aufweisen. Überraschenderweise weisen sie sogar geringere Pigmentdicken auf als die PVD-Aluminiumpigmente "Metalure A" der Fa. Eckart gemäß Vergleichsbeispiel 18.
Der Span der Dickenverteilung ist bei den erfindungsgemäßen Pigmenten vergleichbar mit den PVD-Aluminiumpigmenten. Dies war bisher nicht aus einer Naßvermahlung zugänglich. Das konventionelle Goldbronzepigment aus der Naßmahlung (Vergleichsbeispiel 17) zeigt einen deutlich höheren Span.

Weiterhin ist aus **Tab. 5** ersichtlich, dass die erfindungsgemäßen Messingpigmente gemäß Beispiel 15 eine wesentlich engere Dickenverteilung (Span) als die konventionellen Goldbronzepigmente des Vergleichsbeispiels 17 aufweisen. Zudem weisen die erfindungsgemäßen Messingpigmente gemäß Beispiel 15 und 16 eine geringere Größe d₉₀ als die Pigmente der Vergleichsbeispiele 17 und 18 auf.

Zur weiteren Charakterisierung der erfindungsgemäßen Messingpigmente wurden sogenannte Konterapplikationen auf transparenten Folien erstellt. Hierzu wurde eine MELINEX 400 Folie (PET-Folie, 50 µm) mit einer Tiefdruckfarbe auf Basis eines kommerziell gehandelten Polyvinylbutyrals (PVB) und einer Mischung aus Methoxypropanol und Ethylacetat mittels einer Druckmaschine bedruckt.

Die pigmentierten Folienkonterapplikationen wurden optisch durch eine Glanzmessung bei 60° in Anlehnung an DIN 67 530 (Gerät: micro-TRI-gloss von Byk-Gardner, D-82538 Geretsried, Deutschland) charakterisiert. Kalibriert wurde mittels Dunkelkalibrierung sowie einer schwarzen Spiegelglasplatte mit Werten von 92 für 60°.

Die Farbdichte wurde mit einem Densitometer (Gerät: Densitometer, Fa. X-Rite, D-63263 Neu-Isenburg) gemessen. Kalibriert wurde mit Hilfe eines Weißstandards und des unbedruckten Substrats bei einer Wellenlänge im gelben Bereich.

### Farbdichte = - Ig Remission

Dabei werden die betrachteten Oberflächen in Aufsicht vermessen.

Die auf Grundlage von Druckmaschinenandrucken (Druckmaschine: Rotova 300, Fa. Rotocolor, 3 Farbwerke; Druckgeschwindigkeit 75 m/min, Viskosität 15 s DIN-4-Auslaufbecher, 60, 70, 80 und 90 Linien/cm; Pigmentierungshöhe 25%) ermittelten optischen Eigenschaften von mit erfindungsgemäßen Messingpigmenten gemäß Beispiel 15 und konventionellen Goldbronzepigmenten gemäß Vergleichsbeispiel 17 sowie eingefärbten, herkömmlichen PVD-Aluminiumpigmenten gemäß Vergleichsbeispiel 18 pigmentierten Folienkonterapplikationen sind in nachfolgender **Tab. 6** dargestellt.

Dabei wurde bei Vergleichsbeispiel 18 die Druckfarbe ULTRASTAR (Fa. Eckart) mit zwei unterschiedlichen Konzentrationen einer Mischung von gelben (Yellow 79) und orangen (Solvent Orange 41) Farbstoffen verwendet (Beispiel 18a und 18b). Die Farbstoffe wurden in Form der UltraStar Toner-Reihe (UltraStar Toner TY-21 und TO-11; Fa. Eckart) vermischt, wobei es sich bei der Tonerreihe jeweils um Dispersionen der Farbstoffe in Methoxypropanol handelte.

**Tab. 6: Optische Charakterisierung Pigmente I**

| **Probe** | **Glanz** | | | | **Farbdichte** | | | |
|---|---|---|---|---|---|---|---|---|
| | **(60°)** | | | | | | | |
| **Linien/cm** | **60 l/cm** | **70 l/cm** | **80 l/cm** | **90 l/cm** | **60 l/cm** | **70 l/cm** | **80 l/cm** | **90 l/cm** |
| Beispiel 15 | 500 | 488 | 440 | 428 | 1,45 | 1,44 | 1,32 | 1,22 |
| Vergleichsbeispiel 17 | 243 | 246 | 232 | 230 | 1,00 | 1,00 | 0,84 | 0,77 |
| Vergleichsbeispiel 18a (mit 35% Toner eingefärbt) | 415 | 408 | 405 | 396 | 1,50 | 1,52 | 1,50 | 1,44 |
| Vergleichsbeispiel 18b (mit 59% Toner eingefärbt) | 210 | 207 | 208 | 209 | 1,48 | 1,86 | 1,94 | 1,80 |

**Tab. 6** zeigt, dass die erfindungsgemäße Messingpigmente gemäß Beispiel 15 enthaltenden Folienkonterapplikationen bei allen Druckvarianten einen höheren Glanz als die mit konventionellen Pigmenten gemäß Vergleichsbeispiele 17 und 18 pigmentierten Folienkonterapplikationen aufweisen.
Gegenüber Vergleichsbeispiel 17 weisen die Applikationen der erfindungsgemäßen Pigmente aus Beispiel 15 zudem eine höhere Farbdichte auf.
Der Glanz der Folienkonterapplikationen von Beispiel 15 war ebenfalls höher als bei den Vergleichsbeispielen 18a und 18b. Die höheren Farbdichte der Vergleichsbeispiele 18a und 18b suggerieren jedoch eine stärkere Farbigkeit dieser Applikationen als bei Beispiel 15. Dies war jedoch tatsächlich nicht der Fall.

Die visuelle Beurteilung des Spiegeleffektes der Folienkonterapplikationen ergab nämlich folgende Ergebnisse:
Beispiel 1: klarer, sehr guter Spiegel
Beispiel 4: Spiegel matt, trüb
Beispiel 5a: farbschwacher, silbriger Spiegel
Beispiel 5b: matter Spiegel

Zur weiteren optischen Charakterisierung wurden die Helligkeiten, die Buntheit und der Bunttonwinkel der pigmentierten Folienkonterapplikationen mit den in nachfolgender **Tab. 7** erfassten Versuchsergebnissen ermittelt. Es wurden Helligkeitsmessungen mit einem kommerziell erhältlichen Gerät der Fa. X-Rite (Lichtquelle. D65, 10° Normalbetrachter) in diffuser Meßgeometrie bei einem Beobachtungswinkel von 8° durchgeführt.
Hierbei wurden exemplarisch die Werte bei 60 l/cm vermessen.

Die in **Tab. 7** erfasste Buntheit **C*** beschreibt die relative Sättigung im Verhältnis zum Referenzweiß, also im Vergleich zu einem bestimmten hellsten Punkt eines Farbraums. Der ebenfalls in **Tab. 7** erfasste Bunttonwinkel **h*** ist der dem Farbton zugeordnete Farbwert, welcher auch als Buntton bezeichnet wird.

**Tab. 7 Optische Charakterisierung Goldbronzepigmente II diffuse Meßgeometrie**

| **Probe** | **L*** | **a*** | **b*** | **C*** | **H*** | **Glanz (60°) 60 l/cm** |
|---|---|---|---|---|---|---|
| Beispiel 15 | 84,5 | 3,0 | 31,5 | 31,6 | 84,5 | 500 |
| Vergleichsbeispiel 17 | 78,8 | 3,6 | 27,9 | 28,2 | 82,7 | 243 |
| Vergleichsbeispiel 18a (mit 35% Toner eingefärbt) | 83,8 | 0,0 | 10,2 | 10,2 | 89,8 | 415 |
| Vergleichsbeispiel 18b (mit 59% Toner eingefärbt) | 77,2 | 0,2 | 27,4 | 27,4 | 89,5 | 210 |

Aus der **Tab. 7** ist zu entnehmen, dass die erfindungsgemäßen Messingpigmente gemäß Beispiel 15 farbintensiver waren als die der Vergleichsbeispiele 17 und 18a und 18b. Diese Messungen entsprachen auch viel mehr dem visuellen Eindruck. Dies bedeutet, dass das Goldbronzepigment aus Beispiel 15 aufgrund seiner geringen Pigmentdicke einen hohen Glanz und zusätzlich aufgrund seiner Eigenfarbe einen hohen Farbwert (Chroma) besaß. Weiterhin ist aus Tab. 7 ersichtlich, dass die optischen Eigenschaften der mit Toner eingefärbten Folienkonterapplikationen gemäß Beispielen 18a und 18b mit der als Farbmittel eingesetzten Tonermenge korrelierten. So wiesen die mehr Farbmittel (Toner) enthaltenden Folienkonterapplikationen gemäß Beispiel 18b zwar ein höheres Chroma (Buntheit C*) aber geringere Helligkeiten L* und einen wesentlich verringerten Glanz (60°) als die weniger Farbstoffe (Toner) enthaltenden Folienkonterapplikationen gemäß Beispiel 18a auf. Offenbar streuen die Farbstoffe zu sehr das Licht und vermindern so den metallischen Effekt. Diese Nachteile können mit den erfindungsgemäßen Metalleffektpigmenten überwunden werden.

Als weiteres Beurteilungskriterium wurde die Haftfestigkeit der pigmentierten Konterapplikationen mittels Tesa-Test (Spaltfestigkeit) ermittelt.

Hierzu wurde ein Klebestreifen fest und ohne Blasen auf die Oberfläche geklebt. Anschließend wurde dieser Klebestreifen wieder abgezogen, so dass der Untergrund nicht beschädigt wurde. Die Spaltfestigkeit wurde anhand eines Notensystems visuell von Note 1 (sehr gut) bis Note 5 (sehr schlecht) beurteilt. Eine schlechte Spaltfestigkeit spiegelt sich in einem entsprechend starken Ausriss aus dem Druck wieder.

Es wurde ermittelt, dass die erfindungsgemäßen Messingpigmente gemäß Beispiel 15 eine bessere Haftfestigkeit (Note 2) als die Goldbronzepigmente gemäß Vergleichsbeispiel 17 (Note 4) und die PVD-Aluminiumpigmente des Vergleichsbeispiels 18 (Note 3) aufweisen.

In einer Gesamtbetrachtung der Versuchsergebnisse ist festzustellen, dass die erfindungsgemäßen Pigmente eine bei konventionellen, durch Trockenvermahlung hergestellten Leafing Goldbronzepigmente bisher nicht erreichte Pigmentcharakteristika, insbesondere hinsichtlich Dicke, Dickenverteilung und Deckkraft, aufweisen. Die mit erfindungsgemäßen Pigmenten pigmentierten Folienkonterapplikationen zeichnen sich durch attraktive koloristische Eigenschaften, insbesondere durch einen goldfarbenen Spiegeleffekt mit einer hohen Farbdichte aus, der bisher nicht mit Farbpigmente enthaltenen PVD-Aluminiumpigmente erzielt werden konnte. Mit erfindungsgemäßen Pigmenten pigmentierte Folienkonterapplikationen besitzen eine hohe Haftfestigkeit. Zudem kann durch das hohe Deckvermögen der erfindungsgemäßen Pigmente deren Einsatzmenge im Anwendungsmedium verringert werden.

### C Beispiele PVD-Effektpigmente

### Beispiel 19:

400 g eines handelsüblichen PVD-Pigments (Metalure L55700, Fa. Eckart) wurden mit 2 g des Additivs aus Bsp. 1a in eine Mühle nach Bsp. 1b mit 6,5 kg Stahlkugeln (Durchmesser 0,8 mm) gegeben und verschlossen. Die Mischung wurde 3 h bei 30 U/min vermahlen. Das Mahlprodukt wurde von den Mahlkörpern getrennt und auf einen Feststoffgehalt von 10 % eingestellt.

### Vergleichsbeispiel 20:

Vermahlung wie in Beispiel 19, jedoch wurde als Schmiermittel 2 g einer gewöhnlichen Mischung aus Stearin- und Ölsäure eingesetzt.

### Vergleichsbeispiel 21:

Kommerziell erhältliches PVD-Pigment Metalure L (Fa. Eckart GmbH), das gemäß Beispiel 19, jedoch ohne jeglichen Zusatz von Schmiermittel bzw. Additiv in einer Mühle behandelt wurde.

Die Charakterisierung erfolgte nach den bereits oben beschriebenen Methoden. Partikelgröße, Glanz (Tiefdruck (NC, Acrylat), Spiegel), Spaltfestigkeit

**Tab. 8: Charakterisierung der Pigmente**

| **Probe** | **Schmiermittel** | **Partikelgrößenverteilung** | | **Prüfung Tiefdruckfarbe Nitrocellulose; Pigmentierungshöhe: 3,8%** | | **Prüfung Spiegelapplikation Pigmentierungshöhe: 3,8 %** |
|---|---|---|---|---|---|---|
| | | d₅₀/ µm Beginn | d₅₀/ µm Ende | Glanz 60° | Deckung | Glanz 60° |
| Beispiel 19 | Beispiel 1a | 11,3 | 8,2 | 274 | gut (2) | 497 |
| Vergleichsbeispiel 20 | Mischung Stearin-/Ölsäure | 11,3 | 10,8 | 258 | mittel (3) | 469 |
| Vergleichsbeispiel 21 | - | 11,3 | 10,1 | 208 | gering (5) | 480 |

### Beispiel 22:

Kommerziell erhältliches PVD-Pigment (Metalure L, Fa. Eckart) wurde mit 3 % eines Additivs gemäß Beispiel 1a vermengt und zu einer Metalleffektpigmentpaste weiterverarbeitet.

### Vergleichsbeispiel 23:

Kommerziell erhältliches PVD-Pigment (Metalure L, Fa. Eckart) ohne Additivzusatz.

**Tab. 9: Charakterisierung der Pigmente**

| **Probe** | **Additiv** | **Prüfung Tiefdruckfarbe Nitrocellulose; Pigmentierungshöhe: 3,8 %** | | **Prüfung Spiegellackapplikation Pigmentierungshöhe: 3,8 %** |
|---|---|---|---|---|
| | | Glanz 60° | Deckung | Glanz 60° |
| Beispiel 22 | Beispiel 1a | 297 | sehr gut (1) | 527 |
| Vergleichsbeispiel 23 | - | 292 | gut (2) | 510 |

Die aufgeführten Beispiele zeigten eine Verbesserung der optischen Eigenschaften auch von PVD-Pigmenten durch die erfindungsgemäßen Additive bzw. Schmierstoffe und deren Einsatz nach bzw. während der Vermahlung.
Bei der Vermahlung der PVD-Pigmente mit den Additiven fand eine Verringerung der mittleren Größe (d₅₀) statt. Trotz dieser Teilchengrößenverringerung verbessert sich der Glanz des Pigmentes in der Tiefdruckfarbe bzw. der Spiegellackapplikation. PVD-Pigmente mit geringeren Teilchengrößen sind für bestimmte Anwendungen wie beispielsweise Tiefdruckfarben vorteilhaft.

## Patentansprüche

1. Metalleffektpigment mit Additiv,
**dadurch gekennzeichnet,**
**dass** das Additiv zumindest teilweise auf dem Metalleffektpigment aufgebracht ist und als Struktureinheiten wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind, wobei Carbonsäure wenigstens eine Polycarbonsäure mit zwei bis acht Carbonsäuregruppen ist.

2. Metalleffektpigment nach Anspruch 1,
**dadurch gekennzeichnet,**
**dass** die Carbonsäure mit dem Polyglykolether über eine Esterbindung oder eine Amidbindung kovalent miteinander verbunden sind.

3. Metalleffektpigment nach einem der vorstehenden Ansprüche,
**dadurch gekennzeichnet,**
**dass** die Carbonsäure gesättigt oder ungesättigt ist.

4. Metalleffektpigment nach Anspruch 2 oder 3,
**dadurch gekennzeichnet,**
**dass** die Polycarbonsäure eine dimerisierte, trimerisierte oder tetramerisierte Fettsäure oder Mischungen dieser Formen ist.

5. Metalleffektpigment nach einem der Ansprüche 2 bis 4,
**dadurch gekennzeichnet,**
**dass** die Polycarbonsäure 10 bis 96, vorzugsweise 12 bis 76, Kohlenstoffatome enthält.

6. Metalleffektpigment nach einem der vorstehenden Ansprüche,
**dadurch gekennzeichnet,**
**dass**
der Polyglykolether die Gruppe R¹-X-(R²-O)_{y}-(R³-O)_{z}-(R⁴-O)ₖ- umfasst, wobei die R²-O-, R³-O- und die R⁴-O-Polyethereinheiten statistisch, alternierend oder als Blockcopolymere angeordnet sein können,
X für O, S, (CO)O, NR^{x} steht, wobei R^{x} gleich H oder ein aliphatischer Rest mit 1 bis 20 Kohlenstoffatomen ist und
R¹ ein linearer oder verzweigter aliphatischer Rest oder araliphatischer oder aromatischer organischer Rest mit 1 bis 30 Kohlenstoffatomen ist und R², R³ und R⁴ gleich oder unabhängig voneinander verschieden sein können und jeweils für einen linearen oder verzweigten aliphatischen organischen Rest oder araliphatischen oder aromatischen organischen Rest mit 1 bis 12 Kohlenstoffatomen stehen und
y, z und k natürliche Zahlen sind und unabhängig voneinander für 0 bis 200 stehen, mit der Maßgabe das y+z+k = 2 bis 600 ist.

7. Metalleffektpigment nach Anspruch 6,
**dadurch gekennzeichnet,**
**dass** das Verhältnis der Anzahl der Ethereinheiten y+z+k zur Anzahl der C-Atome der Carbonsäure oder der Polycarbonsäure sowie ggf. der Kohlenwasserstoffreste, des aliphatischen oder araliphatischen Rests R¹ sowie des aliphatischen Restes R Werte von 0,1 bis 4,0 beträgt.

8. Metalleffektpigment nach einem der vorstehenden Ansprüche,
**dadurch gekennzeichnet,**
**dass** die Carbonsäure teilweise oder vollständig verestert ist.

9. Metalleffektpigment nach Anspruch 8,
**dadurch gekennzeichnet,**
**dass** die Carbonsäure teilweise verestert ist und das Additiv eine Säurezahl von 5 bis 140 mg KOH/g Additiv aufweist.

10. Metalleffektpigment nach einem der der vorstehenden Ansprüche,
**dadurch gekennzeichnet,**
**dass** die Carbonsäure teilweise oder vollständig als Carbonsäuresalz vorliegt.

11. Metalleffektpigment nach einem der vorstehenden Ansprüche,
**dadurch gekennzeichnet,**
**dass** die Metalleffektpigmente in kompaktierter Form, vorzugsweise als Granulat, Pellets, Tabletten, Briketts, Würstchen oder als Paste, vorliegen.

12. Verfahren zur Herstellung eines Metalleffektpigmentes nach einem der vorhergehenden Ansprüche,
**dadurch gekennzeichnet,**
**dass** das Verfahren die folgenden Schritte umfaßt:
a) Vermahlen von Metallpartikeln zu Metalleffektpigmenten in Gegenwart eines Additivs, das als Struktureinheiten wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind und wobei die Carbonsäure wenigstens eine Polycarbonsäure mit zwei bis acht Carbonsäuregruppen ist, und Mahlkörpern sowie optional einer Flüssigphase
b) Abtrennen der in Schritt a) erhaltenen mit dem Additiv versehenen Metalleffektpigmente von den Mahlkörpern und optional der Flüssigphase,
c) optional Kompaktieren der in Schritt b) abgetrennten mit dem Additiv versehenen Metalleffektpigmente.

13. Verfahren nach Anspruch 12,
**dadurch gekennzeichnet,**
**dass** Schritt a) in einer Kugelmühle in Gegenwart von Mahlkörpern, vorzugsweise von sphärischen Mahlkörpern, durchgeführt wird.

14. Verfahren nach Anspruch 12 oder 13,
**dadurch gekennzeichnet,**
**dass** das Kompaktieren mittels Granulieren, Pelletieren, Tablettieren, Brikettieren, Filtrieren, Abpressen und/oder Extrudieren durchgeführt wird.

15. Verwendung von Metalleffektpigmenten nach einem der Ansprüche 1 bis 11 zur Herstellung von Beschichtungszusammensetzungen, insbesondere Lacken, Coatings, Druckfarben, Kunststoffen oder kosmetischen Formulierungen.

16. Beschichtungszusammensetzung,
**dadurch gekennzeichnet,**
**dass** die Beschichtungszusammensetzung ein Metalleffektpigment nach einem der Ansprüche 1 bis 11 enthält.

17. Beschichtungszusammensetzung nach Anspruch 16,
**dadurch gekennzeichnet,**
**dass** die Beschichtungszusammensetzung eine Druckfarbe ist.

## Claims

1. Metal effect pigment with additive,
**characterised in that**
the additive is at least partially applied to the metal effect pigment and comprises at least one carboxylic acid having at least 4 carbon atoms and at least one polyglycol etheras structural units, the carboxylic acid and polyglycol ether being covalently bonded to one another, and the carboxylic acid is at least one polycarboxylic acid having two to eight carboxylic acid groups.

2. Metal effect pigment as claimed in claim 1,
**characterised in that**
the carboxylic acid and polyglycol ether are covalently bonded to one another via an ester bond or an amide bond.

3. Metal effect pigment as claimed in one of the preceding claims,
**characterised in that**
the carboxylic acid is saturated or unsaturated.

4. Metal effect pigment as claimed in claim 2 or 3,
**characterised in that**
the polycarboxylic acid is a dimerised, trimerised or tetramerised fatty acid or mixtures of these forms.

5. Metal effect pigment as claimed in one of claims 2 to 4,
**characterised in that**
the polycarboxylic acid contains 10 to 96, preferably 12 to 76, carbon atoms.

6. Metal effect pigment as claimed in one of the preceding claims,
**characterised in that**
the polyglycol ether comprises the group R¹-X-(R²-O)_{y}-(R³-O)_{z}-(R⁴-O)ₖ-, and the R²O-, R³O- and R⁴O-polyether units may be disposed in a random or alternating arrangement or as block copolymers, where X stands for O, S, (CO)O, NR^{x}, and R^{x} is H or an aliphatic radical having 1 to 20 carbon atoms and
R¹ is a linear or branched aliphatic radical or aliphatic or aromatic organic radical having 1 to 30 carbon atoms and
R², R³ and R⁴ may be identical or, independently of one another, different and respectively stand for a linear or branched aliphatic organic radical or aliphatic or aromatic organic radical having 1 to 12 carbon atoms and
y, z and k are natural numbers and, independently of one another, stand for 0 to 200, with the proviso that y+z+k = 2 to 600.

7. Metal effect pigment as claimed in claim 6,
**characterised in that**
the ratio of the number of ether units y+z+k to the number of C atoms of the carboxylic acid or of the polycarboxylic acid and, optionally, of the hydrocarbon radicals of the aliphatic or araliphatic radical R¹ and the aliphatic radical R is based on values of 0.1 to 4.0.

8. Metal effect pigment as claimed in one of the preceding claims,
**characterised in that**
the carboxylic acid is partially or completely esterified.

9. Metal effect pigment as claimed in claim 8,
**characterised in that**
the carboxylic acid is partially esterified and the additive has an acid number of 5 to 140 mg KOH/g additive.

10. Metal effect pigment as claimed in one of the preceding claims,
**characterised in that**
the carboxylic acid is partially or entirely present as carboxylic acid salt.

11. Metal effect pigment as claimed in one of the preceding claims,
**characterised in that**
the metal effect pigment is present in compacted form, preferably as granulate, pellets, tablets, briquettes, sausages or as paste.

12. Method of producing a metal effect pigment as claimed in one of the preceding claims,
**characterised in that**
the method comprises the following steps:
a) grinding metal effect particles to metal effect pigments in the presence of an additive comprising at least one carboxylic acid having at least 4 carbon atoms and at least one polyglycol ether as structural units, the carboxylic acid and polyglycol ether being covalently bonded to one another, and the carboxylic acid is a polycarboxylic acid with two to eight carboxylic acid groups, and grinding media and, optionally, a liquid phase,
b) separating the metal effect pigments obtained in step a) provided with the additive from the grinding media and optionally the liquid phase,
c) optionally compacting the metal effect pigments obtained in step b) provided with the additive.

13. Method as claimed in claim 12,
**characterised in that**
step a) is carried out in a ball mill in the presence of grinding media, preferably spherical grinding media.

14. Method as claimed in claim 12 or 13,
**characterised in that**
compacting is carried out by means of granulating, pelletizing, tableting, briquetting, filtering, pressing and/or extrusion.

15. Use of metal effect pigments as claimed in one of claims 1 to 11 for producing coating compositions, in particular paints, coatings, printing inks, plastics or cosmetic formulations.

16. Coating composition,
**characterised in that**
the coating composition contains a metal effect pigment as claimed in one of claims 1 to 11.

17. Coating composition as claimed in claim 16,
**characterised in that**
the coating composition is a printing ink.

## Revendications

1. Pigment à effets métallisés comportant un additif, **caractérisé en ce que** l'additif est appliqué au moins partiellement sur le pigment à effets métallisés, et en tant que motifs structuraux comprend au moins un acide carboxylique ayant au moins quatre atomes de carbone, ainsi qu'au moins un polyglycoléther, l'acide carboxylique et le polyglycoléther étant liés l'un à l'autre par une liaison covalente, l'acide carboxylique étant au moins un acide polycarboxylique ayant deux à huit groupes acide carboxylique.

2. Pigment à effets métallisés selon la revendication 1, **caractérisé en ce que** l'acide carboxylique et le polyglycoléther sont liés l'un à l'autre par une liaison covalente par l'intermédiaire d'une liaison ester ou d'une liaison amide.

3. Pigment à effets métallisés selon l'une des revendications précédentes, **caractérisé en ce que** l'acide carboxylique est saturé ou insaturé.

4. Pigment à effets métallisés selon la revendication 2 ou 3, **caractérisé en ce que** l'acide polycarboxylique est un acide gras dimérisé, trimérisé ou tétramérisé, ou des mélanges de ces formes.

5. Pigment à effets métallisés selon l'une des revendications 2 à 4, **caractérisé en ce que** l'acide polycarboxylique contient 10 à 96, de préférence 12 à 76 atomes de carbone.

6. Pigment à effets métallisés selon l'une des revendications précédentes, **caractérisé en ce que** le polyglycoléther comprend le groupe R¹-X-(R²-O)_{Y}-(R³-O)_{z}-(R⁴-O)ₓ-, dans lequel les motifs R²-O-, R³-O- et R⁴-O-polyéther peuvent être disposés selon un arrangement statistique, alterné ou sous forme de copolymères à blocs,
X représente O, S (CO)O, NR^{x}, R^{x} étant H ou un radical aliphatique ayant 1 à 20 atomes de carbone, et
R¹ est un radical aliphatique à chaîne droite ou ramifiée ou un radical organique araliphatique ou aromatique ayant 1 à 30 atomes de carbone, et
R², R³ et R⁴ peuvent être identiques les uns aux autres ou d'une manière indépendante différents les uns des autres, chacun représentant un radical organique aliphatique à chaîne droite ou ramifiée ou un radical organique aromatique ayant 1 à 12 atomes de carbone, et
y, z et k sont des nombres entiers, et valent chacun indépendamment des autres de préférence de 0 à 200, à la condition que y+z+k soit égal à 2 à 600.

7. Pigment à effets métallisés selon la revendication 6, **caractérisé en ce que** le rapport entre le nombre des motifs éther y+z+k et le nombre des atomes de carbone de l'acide carboxylique ou de l'acide polycarboxylique, ainsi qu'éventuellement des radicaux hydrocarbonés, du radical aliphatique ou araliphatique R¹, ainsi que du radical aliphatique R, est de 0,1 à 4,0.

8. Pigment à effets métallisés selon l'une des revendications précédentes, **caractérisé en ce que** l'acide carboxylique est partiellement ou complètement estérifié.

9. Pigment à effets métallisés selon la revendication 8, **caractérisé en ce que** l'acide carboxylique est partiellement estérifié, et que l'additif présente un indice d'acide de 5 à 140 mg KOH/g d'additif.

10. Pigment à effets métallisés selon l'une des revendications précédentes, **caractérisé en ce que** l'acide carboxylique se présente en totalité ou en partie sous forme d'un sel d'un acide carboxylique.

11. Pigment à effets métallisés selon l'une des revendications précédentes, **caractérisé en ce que** les pigments à effets métallisés se présentent sous forme compactée, de préférence sous forme d'un granulé, de pastilles, de tablettes, de briquettes, de cylindres ou d'une pâte.

12. Procédé de fabrication d'un pigment à effets métallisés selon l'une des revendications précédentes, **caractérisé en ce que** le procédé comprend les étapes suivantes :
a) broyage de particules métalliques, pour obtenir des pigments à effets métallisés, en présence d'un additif qui en tant que motifs structuraux comprend au moins un acide carboxylique ayant au moins 4 atomes de carbone, ainsi qu'au moins un polyglycoléther, l'acide carboxylique et le polyglycoléther étant liés l'un à l'autre par une liaison covalente, et l'acide carboxylique étant au moins un acide polycarboxylique ayant 2 à 8 groupes acide carboxylique, ainsi que d'éléments broyeurs et en option d'une phase liquide,
b) séparation des pigments à effets métallisés obtenus dans l'étape a) et pourvus d'un additif, des éléments broyeurs et en option de la phase liquide,
c) en option, compactage des pigments à effets métallisés séparés dans l'étape b) et pourvus de l'additif.

13. Procédé selon la revendication 12, **caractérisé en ce que** l'étape a) est mise en oeuvre dans un broyeur à billes en présence d'éléments broyeurs, de préférence d'éléments broyeurs sphériques.

14. Procédé selon la revendication 12 ou 13, **caractérisé en ce que** le compactage est mis en oeuvre par granulation, pastillage, concrétion, briquettage, filtration, expression et/ou extrusion.

15. Utilisation de pigments à effets métallisés selon l'une des revendications 1 à 11 pour fabriquer des compositions de revêtement, en particulier des vernis, des peintures, des encres d'imprimerie, des plastiques ou des formulations cosmétiques.

16. Composition de revêtement, **caractérisée en ce que** la composition de revêtement contient un pigment à effets métallisés selon l'une des revendications 1 à 11.

17. Composition de revêtement selon la revendication 16, **caractérisée en ce que** la composition de revêtement est une encre d'imprimerie.
